# EUROPEAN PATENT APPLICATION

(11) **EP 2 062 903 A1**
(43) Date of publication of application: **27.05.2009**
(21) Application number: 09002816.8
(22) Date of filing: 18.04.2008
(51) Int. Cl.: C07F 7/18, C07F 9/40, C07F 9/535

(54) **Statin intermediates and process for the preparation of statins**

(30) Priority: 18.04.2007 US 925216; 24.05.2007 US 931926; 21.02.2008 US 66678; 11.03.2008 US 69099
(62) Divisional of application: 08743122.7
(71) Applicant: Teva Pharmaceutical Industries Ltd., 49131 Petah Tiqva (IL)
(72) Inventor: Ponnuswamy, A.E., Chittoor District Andhra Pradesh State (IN); Jaware, Jalinder, Ahmednagar District Maharashtra State (IN); Ranjan, Harish, Paschim Vihar New Delhi 110087 (IN); Kansal, Vinood, Kumar, Haryana (IN)
(74) Representative: Wong, Kit Yee

(57) **Abstract**

Provided are intermediates and process for the preparation of statins, preferably rosuvastatin.

## Description

### CROSS-REFERENCE TO RELATED APPLICATIONS

This application claims the benefit of U.S. Provisional Application No. 60/925,216, filed April 18, 2007; 60/931,926, filed May 24, 2007; 61/066,678, filed February 21, 2008, and 61/069,099, filed March 11, 2008. The contents of which are incorporated herein by reference.

### FIELD OF THE INVENTION

The invention relates to the preparation of chirally pure rosuvastatin intermediates and to the preparation of rosuvastatin and pharmaceutically acceptable salts thereof.

### BACKGROUND OF INVENTION

Rosuvastatin calcium has the chemical name (7-[4-(4-fluorophenyl)-6-isopropyl-2-(N-methyl-N-methylsulfonylamino) pyrimidin-5-yl]-(3R, 5S)-dihydroxy-(E)-6-heptenoic acid calcium salt), and has the following chemical formula: 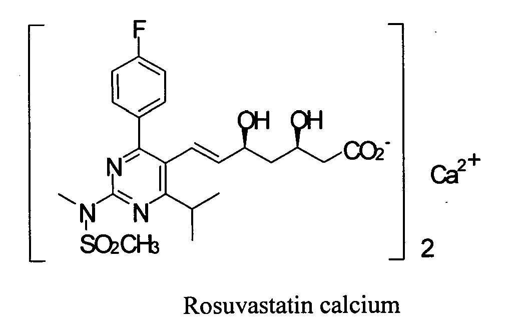 Rosuvastatin calcium is an HMG-CoA reductase inhibitor, developed by Shionogi for the once daily oral treatment of hyperlipidaemia (Ann Rep, Shionogi, 1996; Direct communications, Shionogi, 8 Feb 1999 & 25 Feb 2000). Rosuvastatin calcium is a superstatin, which can lower LDL-cholesterol and triglycerides more effectively than first generation statin drugs.
Rosuvastatin calcium is marketed under the name CRESTOR for the treatment of a mammal such as a human. According to the maker of CRESTOR, it is administered in a daily dose of from about 5 mg to about 40 mg.

U.S. Patent No. 5,260,440 (herein the '440 patent) and PCT publication no. WO 03/097614, disclose the synthesis of rosuvastatin from the late intermediate 3(R)-3(tert-butyldimethylsilyloxy)-5-oxo-6-triphenyl-phophoranylidene hexanoate.

PCT publication no. WO 03/087112 discloses the synthesis of rosuvastatin from a different intermediate, (3R)-3-(t-butyldimethylsilyloxy)-6-dimethoxyphosphinyl-5-oxo-hexanoate, which was synthesized from 3-hydroxy diethyl glutarate via partial hydrolysis by a microorganism to obtain enantiomerically pure glutaric acid derivative according to the following scheme: 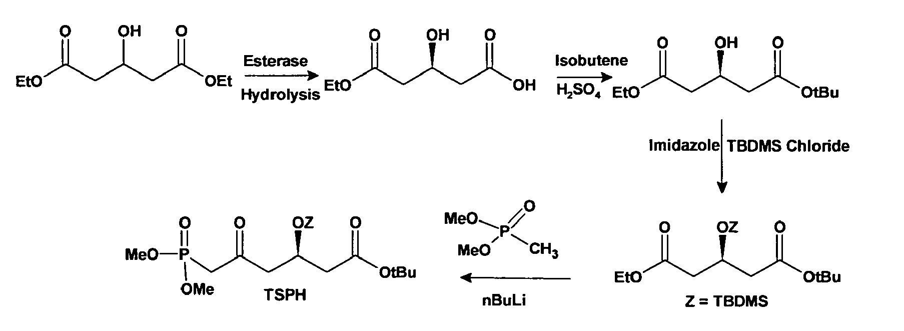 TSPH: (3R)-3-(t-butyldimethylsilyloxy)-6-dimethoxyphosphinyl-5-oxohexanate TBDMS: Tert-butyldimethyl silyl

The process of WO 03/087112 may not be desirable on an industrial scale because it requires the use of an expensive microorganism, such as CLS-BC-14011, during the partial hydrolysis of 3-hydroxy diethyl glutarate. Additionally, the process uses hazardous materials, such as isobutylene gas, during esterification of ethyl-(3S)-3-hydroxyglutaric acid in the presence of sulphuric acid. Also, the purification of TSPH by column chromatography is commercially difficult.

US publication no.: 2005/0070605A1 discloses the enantioselective opening of 3-hydroxy protected glutaric anhydride by phenylethylamine to form an amide bond, and further conversion to obtain the HMG-CoA reductase inhibitor. The process is problematic *inter alia* due to the breaking step of phenyl ethyl amide bond in final step of cerivastatin and in the removal of phenylethylamine in the synthesis of pitavastatin.

PCT publication no. WO 2006/021326 discloses the opening of 3-hydroxy protected glutaric anhydride by methanol to obtain racemic methyl 3(±)-3-(t-butyl dimethylsilyloxy)-6-dimethoxy-phosphinyl-5-oxohexanoate, and the preparation of racemic methyl (3R)-3-(t-butyl dimethylsilyloxy)-6-dimethoxy-phosphinyl-5-oxohexanoate.

US patent no. 5,354,879 discloses the preparation of both methyl (3R)-3-(t-butyl dimethylsilyloxy)-6-dimethoxy-phosphinyl-5-oxohexante and 3(R)-3(t-butyl dimethylsilyloxy)-5-oxo-6-triphenyl-phoporanylidene hexanoate from (3R) 3-[(t-butyl dimethylsilyl)oxy]-glutaric acid 1-(R)-(-)-mandelate and (3S) 3-[(t-butyl dimethylsilyl)oxy]-glutaric acid 1-(S)-(+)-mandelate, respectively. This process employs explosive and toxic materials, such as diazomethane. Moreover, this process is complex and the reported yield is low.

J.Org.Chem.,1991,Vol. 56,3744-3747 discloses the preparation of (3R)-3-(t-butyl dimethylsilyloxy)-6-dimethoxy-phosphinyl-5-oxohexanoate from 3-hydroxy protected glutaric anhydride. In this opening of cyclic anhydride, phenylethylamine is used to prepare amide bond to get enantioselectivity. This process involves hazardous reactions, such as N₂O₄ oxidation, followed by a hydrogenation reaction to cleave the amino group of the resolving agent, and the use of toxic materials such as diazomethane for methylation of the free acid. The process is also expensive due to the use of palladium catalyst. Moreover, the heavy metal could remain as an impurity in the final product and deteriorate its quality.

PCT publication no. WO 00/49014 discloses the synthesis of rosuvastatin using intermediates with other side chains via a Wittig reaction. EP 850,902 discloses the removal of triphenylphosphine derivatives in mixtures.

There remains a need in the art for processes of preparing rosuvastatin that are cost effective, have fewer purification steps, are suitable for industrial scale preparation, result in high yields and are environmental friendly.

### SUMMARY OF THE INVENTION

### Summary of the invention:

One of the embodiments of the present invention provides a salt of a base cation having the following formula (X): 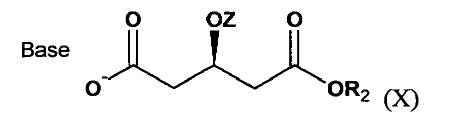 wherein the base is a chiral base;
wherein Z is a hydroxy protecting group and R₂ is an alkyl group of 1-5 carbon atoms.

One of the embodiments of the present invention provides a process for preparing a compound of formula X comprising:
a) reacting a compound of formula IX having the structure: 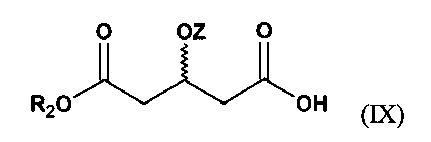 with a chiral base to obtain a salt of the following structure: 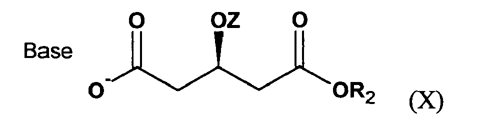
wherein the base is a chiral base;
wherein Z is a hydroxy protecting group and R₂ is an alkyl group of 1-5 carbon atoms.

Another embodiment of the present invention provides a process for preparing the compound of formula: 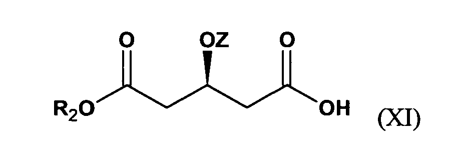 comprising reacting the salt of formula: 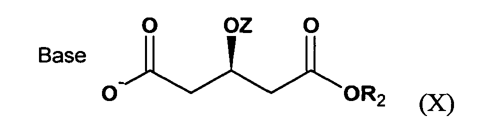 with an acid,
wherein the base is a chiral base,
wherein Z is a hydroxy protecting group and R₂ is an alkyl group of 1-5 carbon atoms.

One of the embodiments of the present invention provides a process for preparing the compound of formula: 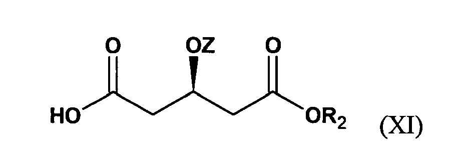
a) reacting a compound of formula IX having the structure: 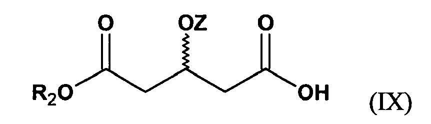 with a chiral base to obtain a salt of the following structure: 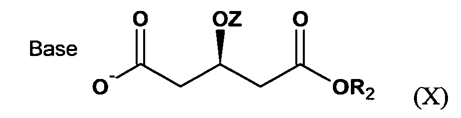 wherein the base is a chiral base;
   wherein Z is a hydroxy protecting group and R₂ is an alkyl group of 1-5 carbon atoms.
b) reacting the salt with an acid.

Still one of the embodiments of the present invention provides a process for the preparation of the compound formula (XII) comprising reacting the compound of formula (XI) 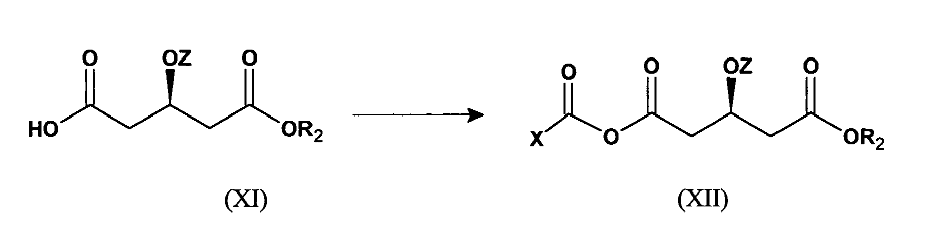 compound of formula XI with a compound of formula R_{f}CO-Y in the presence of base, wherein Y is halogen X is an alkoxy group of C₁ to C₅ carbon atoms or an alkyl group of C₁ to C₅ carbons and R_{f} is a C₁-C₆ alkyl group,

One of the embodiments of the present invention provides a process for preparing the compound of formula (I) from compound of formula (XII) 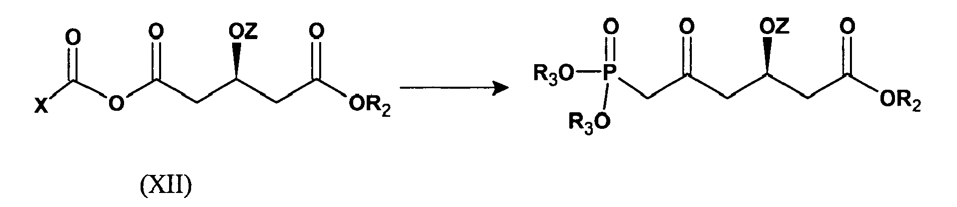 (I)_
comprising reacting dialkyl phosphonate of the general formula: 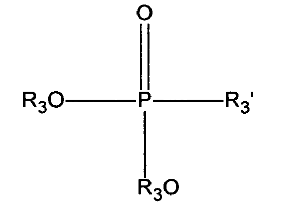 in the presence of base to obtain a phosphonium anion, wherein Z is a hydroxy protecting group; R₂, and R₃ are alkyl of 1-4 carbon atoms; X is an alkoxy group of C₁ to C₅ carbon atoms.

One of the embodiments of the present invention provides a process for preparing a statin comprising the steps of :
a) reacting a chiral base, R(+)-phenylethylamine with a compound of formula IX to obtain a salt of Formula X; 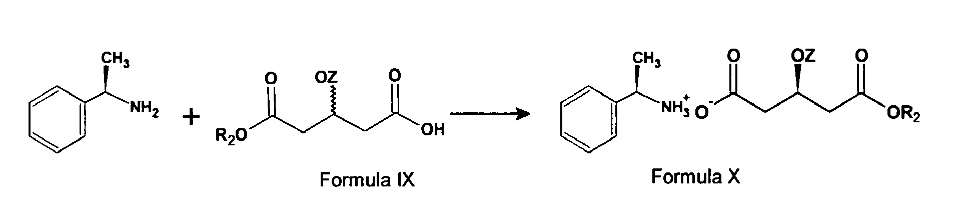
b) reacting compound X with an acid to obtain compound XI:; 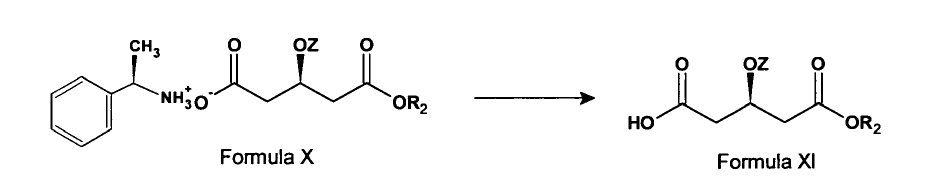
c) reacting compound formula XI with pivaloyl chlorid to obtain compound of formula XII: 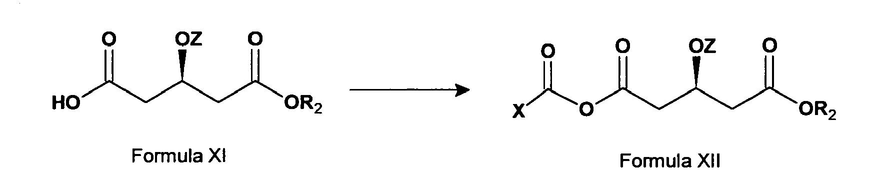
d) reacting compound formula XII with
   i) a phosphonate to obtain a compound of formula I; 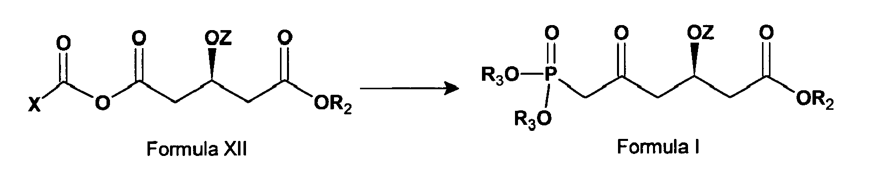
   ii) or alternatively to obtain compound of Formula II 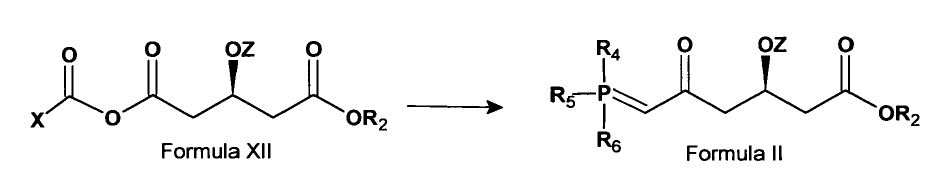
e) converting compound of Formula I or II to a statin.

The present invention relates to processes for the production of chirally pure rosuvastatin intermediates for the preparation of compound of formula (I) and (II), which can be used for the preparation of HMG-CoA reductase inhibitors, said process comprising of the steps of:
a) Reacting a suitable salt at a reaction temperature of about -60°C to 80°C, with the chiral base of the compound of the formula (IX) to obtain the compound of the formula (X);
b) breaking compound of the formula (X) of step (a) in the presence of mineral acid at a reaction temperature of about 0°C to 50°C, to obtain the compound of the formula (XI);
c) reacting the compound of formula (XI) of step (b) with alkyl chloroformate or pivaloyl chloride in the presence of base and in a reaction temperature of -50°C to 0°C, to obtain the compound of the formula (XII),which is an intermediate for the preparation of compound of formula (I) and (II)

The present invention also provides a process for the preparation of the compound of formula (I), comprising reacting compound of formula (A) with dialkyl phosphonate in the presence of base with alkali or alkaline earth metal halide.

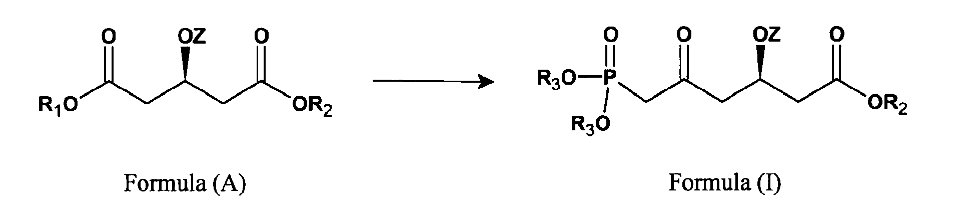

Yet another embodiment of invention provides a chirally pure compound of formula (X). The present invention also provides the preparation of the compound of (XII).

### DETAILED DESCRIPTION OF THE INVENTION

As used herein, the term 'alkyl' refers to a straight or branched hydrocarbon chain radical consisting of carbon and hydrogen atoms, containing no unsaturation, having from one to eight carbon atoms, and which is attached to the rest of the molecule by a single bond, e.g., methyl, ethyl, n-propyl, 1-methylethyl (isopropyl), n-butyl, n-pentyl, 1,1-dimethylethyl (t-butyl), and the like. Preferably the alkyl group is a C₁-C₄ group. Preferably the alkyl group is a C₁-C₄ group, i.e. lower alkyl.

As used herein, the term "alkoxy" denotes alkyl group as defined above attached via oxygen linkage to the rest of the molecule. Representative examples of those groups are - OCH₃, -OC₂H₅ and the like. Preferably the alkoxy group is a C₁-C₄ group.

The substituents in the "substituted alkyl" or "substituted aryl or phenyl" may be selected from the groups such as hydroxy, carboxyl, alkyl (such as C₁-C₄), alkoxy (such as C₆-C₁₂), aryl (such as C₆-C₁₂), arylalkyl (such as C₆-C₁₂), cycloalkyl (such as C₆-C₁₂) and amino.

The present invention provides a process for the preparation of the compound of the formula (I) having the following structure: 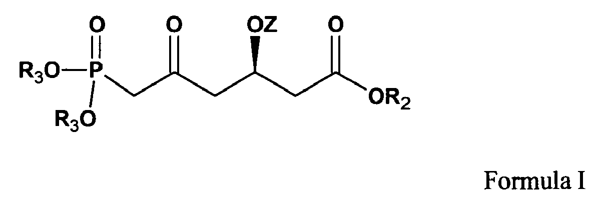 through the intermediate R (+) or S (-) phenyl ethylamine salt of tert-butyl hydrogen 3-hydroxy diethyl glutarate, and chiral purification by crystallization, resulting in high optical purity of the glutaric acid derivative. One aspect of the present invention is the use of alkali and alkali earth metal halide in the process of preparation of the compound of the formula (I), which results in achieving the final compound with less amount of elimination impurity. Another aspect of the invention also relates to a process for the preparation of a chirally pure compound of formula (I) or formula (II) having the following structure: 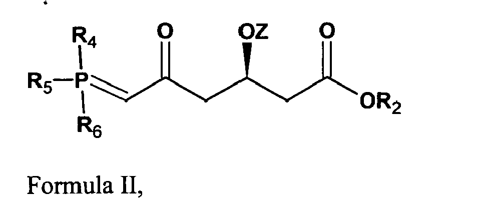 utilizing the compound of the formula (XII) having the following structure:

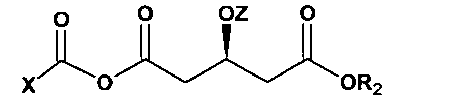

The invention relates to processes for the production of chirally pure compounds of formula (I) and formula (II), which can be used for the preparation ofHMG-CoA reductase inhibitors, 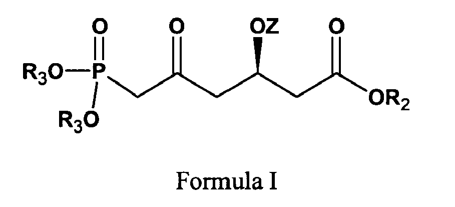 and 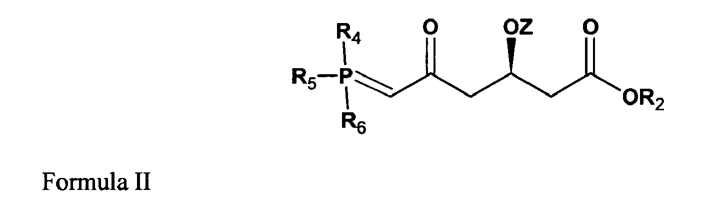 The compounds of formula (I) and formula (II) can be made by a process starting from Formula (IV). Generally, the compound of the formula (IV) maybe utilized as a starting material for the process of preparation of the compound of formula (V) by hydrolyzing the compound of formula (IV) in the presence of base as shown below: 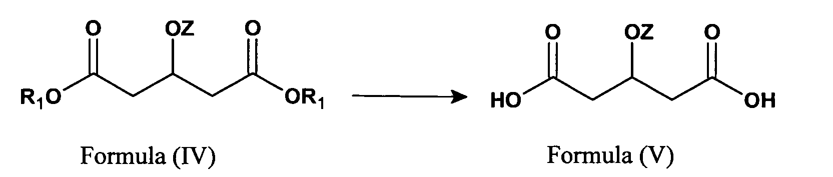 wherein Z is a hydroxy protecting group, and R₁ is an optionally substituted C₁-C₄ alkyl group. The protecting group is preferably a silyl group, including trialkylsilysl group having the formula-Si(A)₃ where each A is independently selected from a C₁-C₆ linear or branched aliphatic or aromatic group. Examples of silyl groups include triethylsilyl, triisopropylsilyl, tert-butyldiphenylsilyl, tert-butyldimethylsilyl. The silyl group is most preferably tert-butyldimethylsilyl. Example of bases include alkali metal and alkali earth metal bases, including hydroxides, carbonates and bicarbonates of alkali or alkali earth metals. Preferred bases are potassium hydroxide or sodium hydroxide and most preferably sodium hydroxide. The process may further comprise a solvent. A suitable solvent that can be used is a C₁-C₄ alcohol. Preferably the solvent for the hydrolysis is methanol. The temperature can be of about 30°C to about 120°C.

As exemplified, compound of formula IV is combined with a suitable solvent, such as methanol, water and a base, such as sodium hydroxide. A preferred temperature range is about 30°C to about 130°C. The hydrolysis reaction is complete in about 1-2 hours. A white colored salt can be observed. An acid is then added to convert the salt of compound V to compound V. Hydrochloric acid or sulfuric acid can be used. The reaction mixture can then be extracted with a water immiscible organic solvent such as dichloromethane. The organic solvent can be evaporated, such as under a pressure of less than one atmosphere, or a temperature of about 25°C to obtain a crude product. A suitable drying temperature is about 40°C to about 50°C. The crude product can be slurried in a hydrocarbon, such as hexane to obtain a more pure product. The pure product can then be dried at elevated temperature, such as about 40°C to about 60°C.

An isomer of compound of Formula IV is disclosed in W02003/087112, incorporated herein by reference.

The compound of the formula (VI) maybe prepared by cyclization of the of the compound of formula (V) in the presence of aliphatic acid anhydride as shown below: 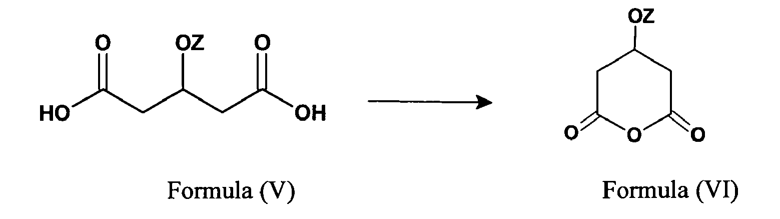 wherein Z is a hydroxy protecting group as described above. The aliphatic acid anhydride can have the following structure: 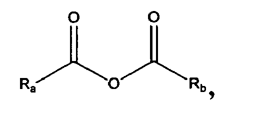 wherein Rₐ and R_{b} are independently selected from C₁-C₅ alkyl groups. Examples of acid anhydrides include C₃ to C₉ aliphatic acid anhydride. Examples of such acid anhydrides include formic acid anhydride, acetic acid anhydride, propionic acid anhydride, butyric acid anhydride, and mixtures, and most preferably acetic anhydride. The temperature can be of about 40°C to about 60°C.

As exemplified, compound of Formula IV can be combined with an anhydride, such as acetic anhydride. The reaction mixture can then be heated, such as about 100°C to about reflux temperature to accelerate the reaction. The reaction is generally complete after about 1-3 hours. The remaining acetic anhydride can be removed by reducing the pressure to less than one atmosphere and/or heating, such as to a temperature of about 75-80°C. The crude product can be crystallized from a C₅-C₁₂ hydrocarbon such as a cyclic or linear hexane. The pure product can be dried, such at under reduced pressure and/or elevated temperature of about 30°C to about 50°C.

The compound of formula (VII) where the squiggly line represents a racemic mixture of isomers maybe prepared by comprising opening of the compound of formula (VII) in the presence of alcohol as shown below: 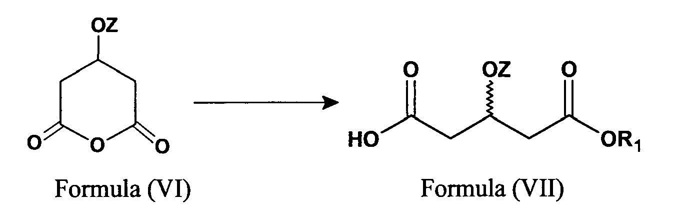 wherein Z and R₁ wherein R1 is a C₁-C₅ alkyl group (R₁ Is preferably methyl). The alcohols used have the formula R₁-OH, wherein R₁ is a C₁-C₅ group. Examples of alcohols include C1-C5 alcohols such as methanol, ethanol, propanol, isopropanol, n-butanol, iso-butanol, tert-butanol, pentanol, and most preferably methanol. Example of bases include alkali metal bases and alkaline earth metal bases, such as hydroxides, carbonates and bicarbonates, more particularly potassium hydroxide or sodium hydroxide and most preferably sodium hydroxide. A base in not necessary when methanol is used as a solvent.

As exemplified, compound VI is combined with a C₁-C₅ alcohol such as methanol, and the reaction mixture is heated to accelerate the reaction. Heating can be carried out to a temperature of about 60°C to about 65°C. The product can then extracted with a water immiscible organic solvent, such as dichloromethane. The product can be recovered by removing the solvent (e.g. dichloromethane), such as by evaporation.

Yamaguchi esterification of the compound of formula (VII) is carried out in the presence of alcohol having the formula R2-OH with base and optionally a coupling agent yields the compound of formula VIII, as shown below:. 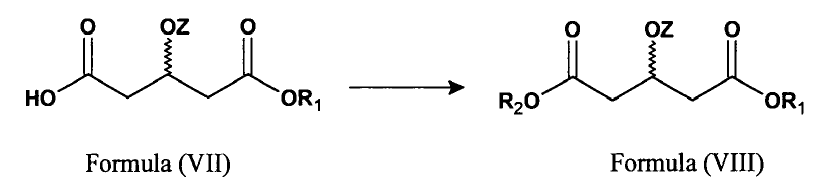 wherein Z is as described above and R₁ and R₂ are, independently, optionally substituted alkyl of 1-4 carbon atoms. Examples of alcohols having the formula R2-OH include alcohol where R2 is a C₁-C₅ alcohol, such as methanol, ethanol, propanol, isopropanol, n-butanol, iso-butanol, tert-butanol, pentanol, and most preferably methanol. Bases such as organic amines, both aryl and alkyl amines, and nitrogen heterocycles optionally substituted with aryl and alkyl groups can be used. On example of a base is DMAP. Amines of formula NR₃ preferably wherein the groups (R) are independently selected from C₁₋₆ alkyl and C₆-Cl₂ aryl. R₁ and R₂ are preferably different from each other to allow for selective hydrolysis of compound of Formula VIII. Preferably R₂ is a t-butyl group and R₁ is a methyl group.

As exemplified, compound of Formula (VII) can be combined with a solvent such as dichloromethane and cooled. Then a coupling agent such as DCC, t-butanol or another alcohol, and a base catalyst such as dimethylaminopyridine are combined with the reaction mixture. The reaction mixture is then stirred to obtain compound of formula VIII. The reaction mixture can then be evaporated and the residue dissolved in a hydrocarbon such as toluene. The toluene can then be evaporated to obtain a residue.

The compound formula (IX) is prepared by selective hydrolysis of the compound of formula (VIII) in the presence of base, as shown below: 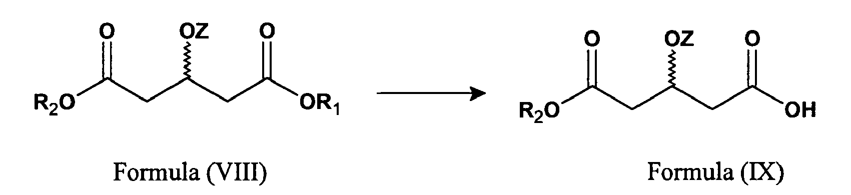 wherein Z, R₁ and R₂ are as described above. Example of bases include alkali metal, alkali earth metal, more particularly potassium hydroxide or sodium hydroxide and most preferably sodium hydroxide. The reaction can be carried out in the following solvents: C₁-C₄ alcohols. The temperature can be of about 35°C to about 120°C.

As exemplified, compound of Formula VIII where R₂ is a t-butyl group and R₁ is a methyl group is combined with a C₁-C₄ alcohol such as ethanol and an alkali metal or alkaline earth metal base such as sodium or potassium hydroxide. The reaction mixture is heated to accelerate the reaction, such as to a temperature of about 35C to about 120C , preferably 40°C to about 60°C. Batchwise addition of the base can be used to avoid diacid formation (such as 4% solution in 1-1.2 molar equivalents in lot wise, slow addition). The starting material preferably has a t-butyl ester and a methyl ester, where the methyl ester is hydrolyzed but not the t-butyl ester. After the reaction, water can be added to the reaction mixture followed by a water immiscible solvent such as toluene. The aqueous layer can be acidified to move the organic compound to the toluene layer, from which it can be recovered by evaporating the toluene.

The present invention also provides a salt having the following formula: 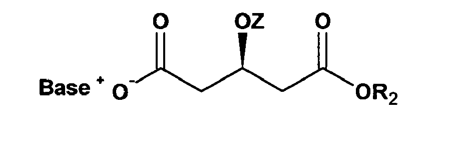 wherein the base is a chiral base;
wherein Z is a hydroxy protecting group and R₂ is an alkyl group of 1-5 carbon atoms. Preferably, the chiral base is an amine. Preferably R₂ is t-butyl. With chiral amine bases, the salt may have has the following structure: 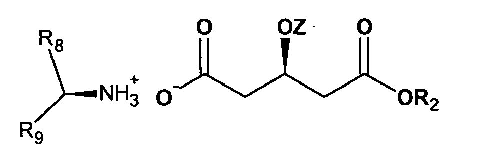 wherein Z and R₂ are the same as described above, wherein R₈ and R₉ are different and each is independently selected from the group consisting of C₁₋₁₅ alkyl, and C₆ to C₁₂ aryl. Preferably R₈ is C₁₋₁₅ alkyl, more preferably C₁-C₄ alkyl and R₉ is a C₆ to C₁₂ aryl, more preferably phenyl. Most preferably R₈ is methyl and R₉ is phenyl.

Preferably, the base is a phenylalkylamine. More preferably, the phenylalkylamine is selected from a group of 1-phenylethylamine, 1-phenylpropylamine, 1-phenylisobutylamine, 1-phenylbutylamine and 1-phenylpentylamine. Most preferably the amine is R (+)-phenylethylamine or S (-)-phenylethylamine. The R (+)-phenylethylamine salt has the following structure:

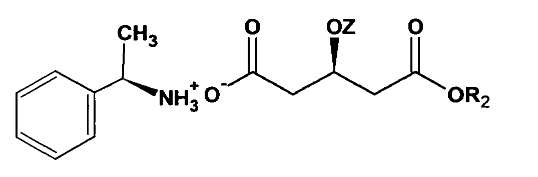

### Compound X

The above salts can be prepared by combining the compound of Formula IX with a chiral base as described above, according to the following scheme (shown from general compounds to more specific compounds): 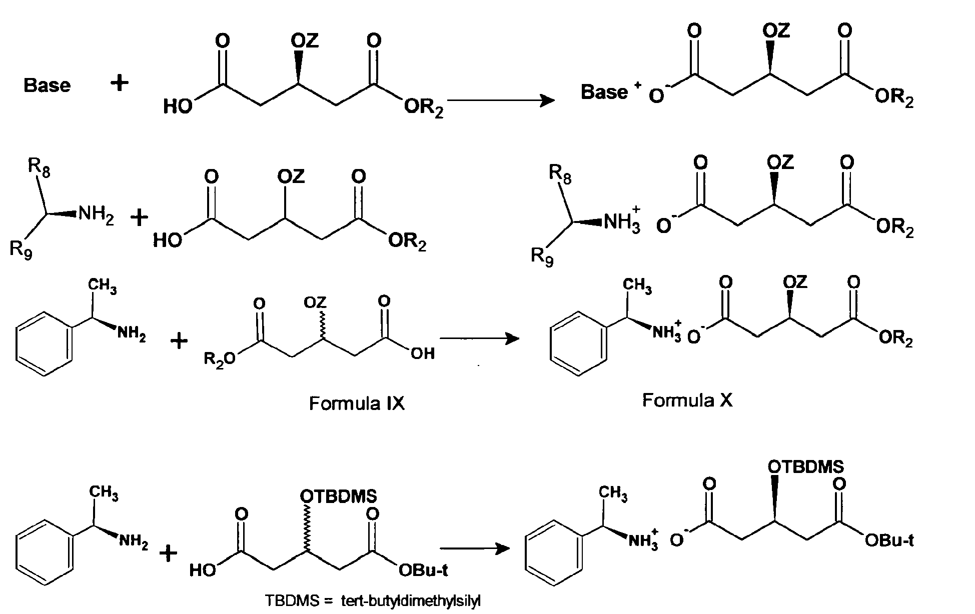 (R8 and R9 are groups as described above). Preferably the base is R (+)-phenylethylamine or S (-)-phenylethylamine, most preferably R (+)-phenylethylamine.

Preferably, the chiral base is in the amount of 1-2 molar equivalents based on the compound of formula (IX).

The reaction can be carried out in a solvent selected from the group of C₁-C₄ aliphatic alcoholic solvents, such as methanol, ethanol, isopropyl alcohol, n-butanol, and t-butyl alcohol, preferably isopropyl alcohol; C₆- C₁₀ aromatic and aliphatic hydrocarbons, such as toluene, benzene, hexanes and cyclohexane; C₂- C₈ aliphatic esters, such as methyl acetate, ethylacetate, t-butylacetate and isopropyl acetate; C₄-C₈ ethers, such as methyl t-butyl ether and tetrahydrofuran; and chlorinated solvent (C₁-C₆ alkyl substituted with 1-4 chlorine atoms) such as dichloromethane, dichloroethane, chloroform, and carbon tetrachloride, tetrachloroethylene, and tetrachloroethane. Preferably the solvent is isopropanol.

The reaction temperature for formation of the salt is preferably about -60°C to 80°C, more preferably about 0°C to 70°C. If the temperature rises above about 30°C a low yield may result; if the temperature of the reaction is below about 0°C, the reaction rate slows down. Preferably the temperature for salt formation is about 0°C to about 30°C.

As exemplified, compound IX is combined with a suitable solvent such as a C₁-C₅ alcohol, preferably isopropanol, and a chiral amine base, preferably, R(+) phenylethyl amine. The reaction mixture can be maintained until observing a white colored salt. The reaction mixture can be heated, after observing the salt such as to a temperature of about 60°C to about 70°C, to obtain a solution, followed by crystallization. Crystallization can be carried out by cooling to a temperature of about 10°C to about 30°C. The product can then be dried. Drying can be carried out at a pressure of less than one atmosphere or at elevated temperature, such as about 30°C to about 50°C. The chiral purification process can be repeated multiple times as illustrated in example 7 where the oily residue obtained from the first chiral purification is dissolved in an alcohol and the process is repeated.

The isomer of compound IX remaining in the mother liquor can be further recycled. This isomer has the following structure: 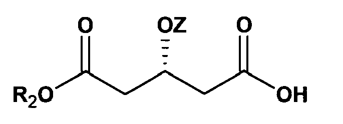 The undesired isomer can be recycled by racemisation, for example, by reaction with a base such as an alkali metal or an alkaline earth metal hydroxide, including sodium hydroxide.

As exemplified, the mother liquor containing the isomer can be extracted with a solvent, such as a C₆-C₁₂ aromatic hydrocarbon, preferably toluene, particularly at acidic pH. A C₁-C₄ alcohol, preferably under dry conditions, can be added to the toluene. A base is added to racemize the isomer. The temperature can be raised to accelerate the reaction, such as of about 30°C to about 50°C. The racemized product can then be acidified to convert the salt to a free acid. It can be extracted with an organic solvent such as dichloromethane. The product can then be recovered as a residue by removing the solvent.

One embodiment of the invention comprises a process for the preparation of the compound formula (XI) comprising reacting the salt of structure 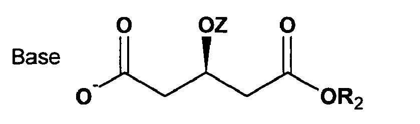 with an acid such as hydrochloric acid. The salt can be that formed with a chiral amine base.
The following illustrates the scheme (shown with general compounds (top) and with more specific compounds): 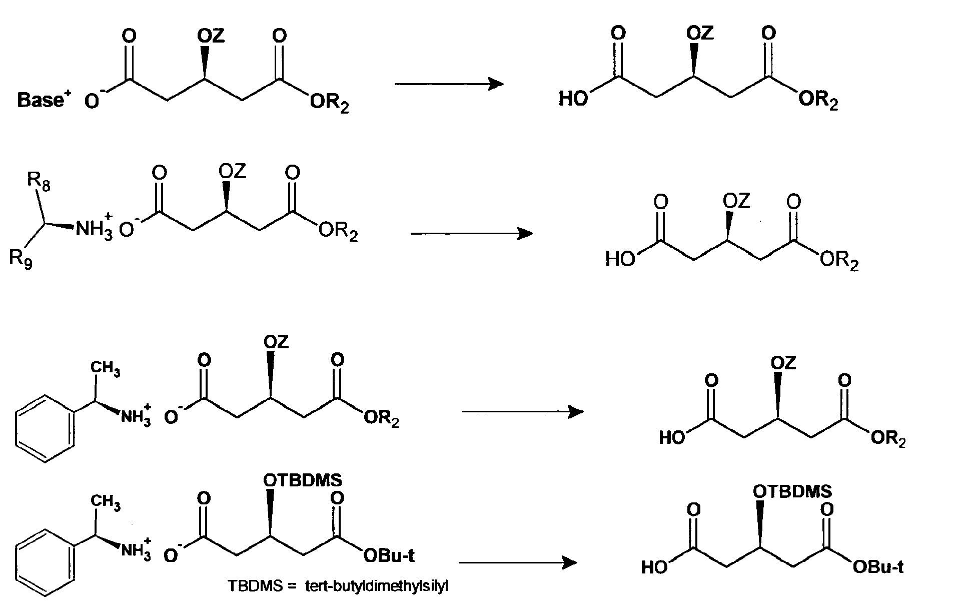 by treating the salt with an acid, wherein Z is a hydroxy protecting group and R₂ is an optionally substituted alkyl of 1-5 carbon atoms. The hydroxy protecting group Z is preferably a silyl group, including trialkylsilyl group having the formula-Si(A)₃ where each A is independently selected from a C1-C6 linear or branched aliphatic or aromatic group. Examples of silyl groups include triethylsilyl, triisopropylsilyl, tert-butyldiphenylsilyl, tert-butyldimethylsilyl. The silyl group is most preferably tert-butyldimethylsilyl.

The reaction preferably takes place (including with other salts including Xa and Xb) in the presence of a reaction solvent. Preferably the solvent is a water miscible solvent. Water is preferably used as a reaction solvent.

Suitable acids include, for example, hydrochloric acid and acetic acid, preferably in aqueous solution. C1-C8 aliphatic organic acids such as formic and acetic acids can also be used. Diluted hydrochloride acid can be used in a concentration range of 1N to 10N more preferably 1N-2N and dilute acetic acid can be used in a range of 5% - 10%. Preferably, dilute hydrochloric acid is used in an amount of about 1-2 equivalents based on the compound of formula (X). The reaction temperature is preferably about 0°C to 50°C, more preferably about 0°C -30°C.

As exemplified, compound X is combined with a salt such as sodium chloride to obtain saturation and an acid such as hydrochloric acid. The reaction mixture is stirred and is extracted with a water immiscible organic solvent such as dichloromethane. The solvent can be removed by evaporation to obtain a residue.

The compound of formula XI obtained is enantiomerically pure, preferably at least about 90% enantiomerically pure, more preferably at least about 95% enantiomerically pure and most preferably at least about 99% enantiomerically pure.

The present invention also provides a process for preparing the chiral pure R-isomer compound of formula:, 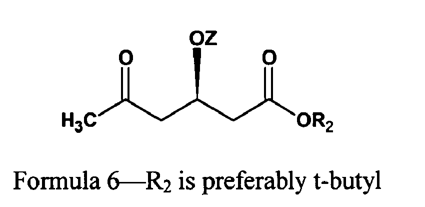 by reacting a racemic mixture having chiral purity of less than desried, such as less than 90%, with a compound (R)-(+)-phenylethylamine of formula 9a, having the following structure: 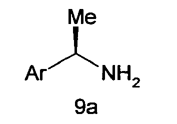 , or a compound (S)-(-)-phenylethylamine of formula 9b, having the following structure: 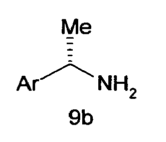 The resolution comprises combining the compound to be purified with chiral ratio of about 80:20 to about 85:15 with compound of formula 9a or 9b to get a salt of formula 8b, having the following structure: 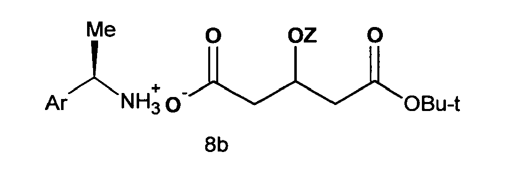 in an aliphatic alcoholic solvent is a C₁-C₄ alcohol selected from the group consisting of: as methanol, ethanol, isopropyl alcohol, n-butyl alcohol and t-butyl alcohol, more preferably isopropyl alcohol. The compound of formula 9a is used in a molar ratio of about 1 to about 2 to the compound of formula 6a at a temperature of about 0°C to about 70°C. The obtained product is crystallized to get chirally pure salt of formula 8c having the following structure: 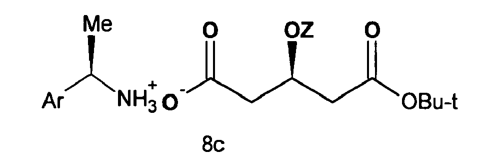 The salt of formula 8c is hydrolyzed in aqueous medium using a mineral acid to get the compound of formula 6 with chiral purity of about 99 to about 100%, more preferably about 99.5 to about 99.8% as measured by chiral HPLC. The mineral acid may be: a dilute hydrochloric acid or dilute sulfuric acid. Preferably dilute hydrochloric acid is used. Hydrochloric acid is added in an amount of about 1 to about 2 equivalents with regard to the compound of formula 8c, at a temperature of about 0°C to about 50°C, preferably at about 0°C to about 30°C.

Yet another embodiment of the invention comprises a process for the preparation of the compound formula (XII) including reacting the compound of formula (XI) with or a compound of formula **R_{f}CO-Y** in the presence of base as shown below: 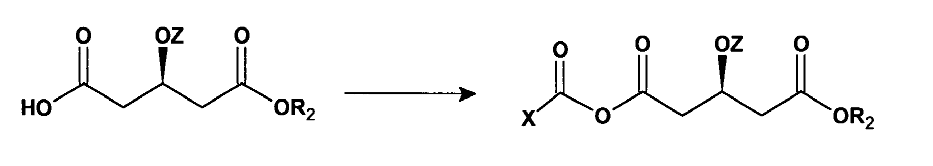 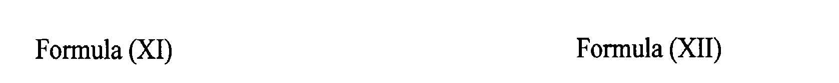 wherein Z is a hydroxy protecting group as described above and R₂ has the same meaning as described above; and X is an alkoxy group of C₁ to C₅ carbon atoms or an optionally substituted alkyl group of C₁ to C₅ carbons, wherein R_{f} refers to a C₁-C₆ alkyl group, Y is halogen. The alkyl chloroformate has the formula RₑOCOCl, wherein Rₑ refers to a C₁-C₆ alkyl group, preferably C₁-C₂. Preferably R_{f} is a C₁-C₆ alkyl group, more preferably C₂-C₄ and most preferably t-butyl, and Y is preferably C₁.

Preferably, the compound of formula R_{f}CO-Y such as pivaloylchloride is used in an amount of about 1-5 equivalents based on the compound of the formula (XI), more preferably 1-2 equivalents based on the compound of the formula (XI).

The reaction solvent can be at least one of C₁- C₄ aliphatic alcoholic solvents, such as methanol, ethanol, isopropyl alcohol, n-butanol, and t-butyl alcohol, preferably isopropyl alcohol; C₆- C₁₀ aromatic and aliphatic hydrocarbons, such as toluene, benzene, hexanes and cyclohexane; C₂- C₈ aliphatic esters, such as methyl acetate, ethylacetate, t-butylacetate and isopropyl acetate; ethers, such as methyl t-butyl ether and tetrahydrofuran; and chlorinated solvent (C₁-C₆ alkyl substituted with 1-4 chlorine atoms) such as dichloromethane, dichloroethane, chloroform, and carbon tetrachloride, tetrachloroethylene, and tetrachloroethane.

As a base, organic amines are preferred. Particularly, amines such as trialkylamine of formula NRₐR_{b}R_{c} preferably wherein Rₐ, R_{b}, and R_{c} are independently selected from C₁₋₆ alkyl group, more preferably C₁₋₄ and most preferably C₂ can be used, preferably in an amount of about 1-5 equivalents based on the compound of formula (XI), more preferably about 1-3 equivalents.

The reaction can be carried out at a temperature range of -50°C to 50°C. If the temperatures rises above about 50°C, by-products may be produced; and further if the temperature of the reaction is below about -50°C, the reaction rate slows down. Preferred temperature ranges of the reaction are -50° to 0°C, and more preferably about -50°C to about 0°C, and most preferably -50°C to -20°C.

In one embodiment, compound of Formula XI is combined with a compound of formula compound of formula R_{f}CO-Y such as pivaloyl chloride and an organic solvent. A C₆- C₁₀ aromatic or aliphatic hydrocarbon such as toluene, or a C1-C6 chlorinated hydrocarbon such as dichloromethene or dichloroethane is suitable. The reaction is then cooled to 50°C to -20°C, to which a base such as a trialkylamines is added. NRₐR_{b}R_{c} preferably wherein Rₐ, R_{b}, and R_{c} are independently selected from C₁₋₆ alkyl group, more preferably C₁₋₄ and most preferably C₂. The temperature I then raised and the reaction is quenched, such as by addition of water. Compound of Formula XII can then be recovered from the organic layer. The organic layer can be separated and evaporated, such as under pressure of less than one atmosphere to obtain compound of Formula XI as a residue.

The compound of formula (I) can be prepared by reacting the compound of formula (XII) with a salt of dialkyl alkylphosphonate of the general formula: 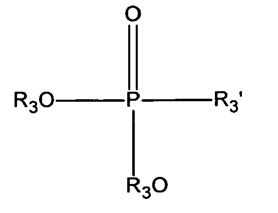 as shown below: 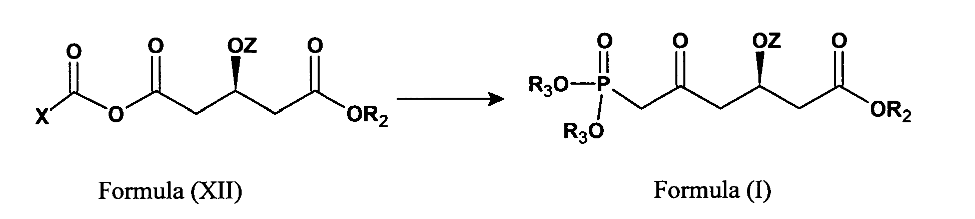 , wherein Z is a hydroxy protecting group, as described above; R₂, and R₃ are, independently, an optionally substituted alkyl of 1-4 carbon atoms; X is an alkoxy group of C₁ to C₅ carbon atoms or an optionally substituted alkyl group of C₁- C₅ carbons.. Preferably R₃ and R₃' are methyl groups. To form the Wittig reagent (ylide), the phosphonate depicted above is suspended in a solvent such as diethyl ether or THF (tetrahydrofuran) and a strong base, preferably a C₁- C₈ aryl or alkyl metal base, such as the organolithium reagents phenyllithium or n-butyllithium is added. The lithium salt has the following structure:

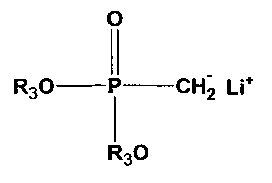

The process can includes the steps of: a) combining a first solution containing C₁- C₈ alkyl or aryl metals such as an alkyllithium, e.g. n-butyl lithium or phenyllithium with a mixture containing dialkyl phosphonate and a solvent such as tetrahydrofuran to form a reaction mixture, and b) adding a second solution containing the compound of formula (XII) to the reaction mixture obtained in step a). The solution containing the compound of formula (XII) is preferably added to the reaction mixture over a period of about 5 to 90 minutes. The reaction can be carried out at a temperature range of -110°C to 0°C. However, if the temperature rises above about -50°C, by-products may be produced; and further if the temperature of the reaction is below about -110°C, the reaction rate slows down. Therefore, the optimal temperature range of the reaction is -110° to -50°C.

Preferably, a C₁-C₄ dialkyl phosphonate is used, and more preferably dimethyl methylphosphonate is used in the process of the invention, preferably in an amount of about 1-5 equivalents based on the compound of the formula (XII), more preferably 2-4 equivalents.

Preferably, the solvent is selected from C₁- C₄ aliphatic alcoholic solvent, a C₆- C₁₀ aromatic and C₅-C₈ aliphatic hydrocarbon, a C₂- C₈ aliphatic ester, a C₄-C₈ ether (including cyclic compounds), and a C1-C6 aliphatic solvent with one, two or three chlorine atoms. Examples of such solvents include toluene, benzene, xylene, cyclohexane; ethers, methyl t-butyl ether, tetrahydrofuran and tetrahydrofuran. Preferably the solvent is tetrahydrofuran. Suitable bases include C₁- C₈ alkyl lithium bases, such as n-butyl lithium, preferably in the amount of 1-5 equivalents based on the compound of formula (XII), more preferably in the amount of 3-4 equivalents.

As exemplified, a compound of formula: 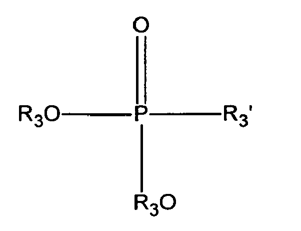 as described above, preferably dimethylmethylphophonate is combined with a suitable solvent such as a C4-C8 ether such as tetrahydrofuran. The reaction mixture is then cooled before addition of a strong base such an organolithium reagent such as phenyllithium or n-butyllithium. The reaction is maintained to obtain anion formation. Compound of Formula XII is then added to the reaction mixture, preferably in the same solvent. The reaction mixture is obtained to complete the reaction. The reaction can be quenched by addition of ammonium chloride. The reaction can be carried out at a preferred temperature of about - 70C to about -80C. Afterwards, the reaction mixture can be warmed, such as to about 25C, and the pH adjusted to about 5 to about 6 with an acid such as HCl. The product, compound of formula one can be extracted into a water immiscible solvent such as toluene. The toluene can then be evaporated to obtain the product.

The compound of formula (XII) can also be utilized for the preparation of the Formula (II) by reacting the compound of formula (XII) with triarylphosphonium alkylhalide of the general formula: 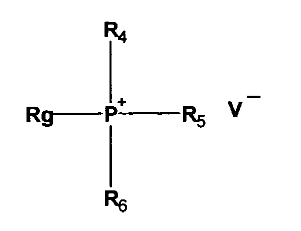 in the presence of base, as shown below: 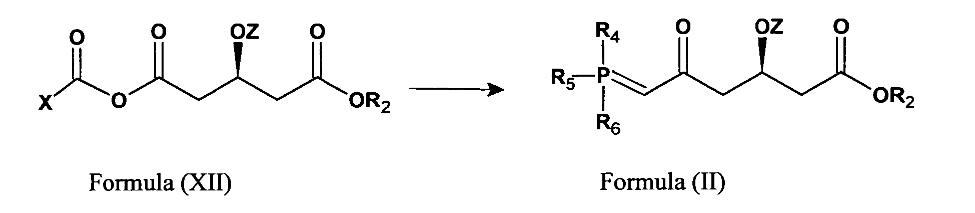 wherein Z is a hydroxy protecting group, as described above and R₂ is optionally substituted alkyl of 1-4 carbon atoms; X is an alkoxy group of C₁ to C₅ carbon atoms or an optionally substituted alkyl group of C₁ to C₅ carbons; R₄, R₅, and R₆, independently, are selected from from C₆-C₁₀ aryl group, preferably phenyl or substituted phenyl groups and R_{g} is a C1-C6 group, wherein V is halide, e.g., Cl, Br, I, preferably Br. Preferably the triarylphosphonium alkylhalide is triphenylphosphonium methylbromide. Preferably the triarylphosphonium alkylhalide such as triphenylphosphonium methylbromide, is present in an amount of about 1-5 equivalents based on the compound of the formula (XII), more preferably 2-4 equivalents.

The process can include the steps of: a) combining a first solution containing n-butyl lithium with a mixture containing dialkyl phosphonate and tetrahydrofuran to form a reaction mixture, and b) adding a second solution containing the compound of formula (XII) to the reaction mixture obtained in step a), at a temperature of about -55°C to -50°C. The solution containing the compound of formula (XII) is preferably added to the reaction mixture over a period of about 30 to 120 minutes. The reaction temperature is preferably maintained at about -55°C to -50°C.

Preferably, the solvent is selected from C₁- C₄ aliphatic alcoholic solvent, a C₆- C₁₀ aromatic and C₅-C₈ aliphatic hydrocarbon, a C₂- C₈ aliphatic ester, a C₄-C₈ ether (including cyclic compounds), and a C1-C6 aliphatic solvent with one, two or three chlorine atoms. Specific examples of such solvents include aliphatic and aromatic hydrocarbons, such as toluene, benzene, xylene, and cyclohexane; and ethers, such as methyl t-butyl ether and tetrahydrofuran, preferably tetrahydrofuran. The C1-C8 alkyllithium such as N-butyl lithium or other C₁- C₈ alkyl metal can be used as a base, preferably in the amount of 1-5 equivalents based on the compound of formula (XII), more preferably in the amount of 3-4 equivalents.

The compound of formula (I) can also be prepared from the compound of Formula A by reacting compound of formula (A) with dialkyl phosphonate in the presence of base with alkali or alkaline earth metal halide. 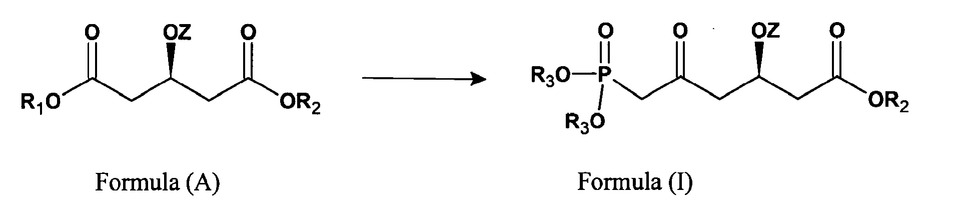 wherein Z is the same as described above and R₂ and R₃ are, independently, optionally substituted alkyl of 1-5 carbon atoms, preferably C₁-C₄.

Preferably, a C₁ to C₄ dialkyl phosphonate is used, more preferably, dimethyl methylphosphonate is used. Preferably, dialkyl phosphonate is used in the amount of 1-5 equivalents based on the compound of the formula (A), more preferably 2-5 equivalents is used.

Optionally, the reaction takes place in a solvent. Preferably, the solvent is selected from C₁- C₄ aliphatic alcoholic solvent, a C₆- C₁₀ aromatic and C₅-C₈ aliphatic hydrocarbon, a C₂- C₈ aliphatic ester, a C₄-C₈ ether (including cyclic compounds), and a C1-C6 aliphatic solvent with one, two or three chlorine atoms. Examples of such solvents include toluene, benzene, xylene, hexane, cyclohexane, methyl t-butyl ether, tetrahydrofuran , and mixtures thereof. Preferably, the solvent is tetrahydrofuran.

Preferably, the base is a C₁ to C₈ alkyl metal. Preferably, the base is n-butyl lithium. Preferably, the base is used in the amount of 1-5 equivalents based on the compound of formula (A), more preferably in the amount of 3-4 equivalents.

Preferably, the catalyst is an alkali or alkaline earth metal halide. Preferably, the alkali metal is lithium. Preferably, the alkaline earth metal is magnesium. Preferably, the catalyst is selected from anhydrous lithium chloride, lithium bromide, lithium iodide, magnesium chloride, magnesium bromide, magnesium iodide, and mixtures thereof. More preferably, the catalyst is anhydrous lithium chloride. Preferably, the catalyst is used in the amount of 0.5-5 equivalents based on the amount of compound of the formula (A).

Optionally, the process comprises adding a first solution comprising a base to a mixture containing dialkyl phosphonate, a catalyst, and a solvent followed by adding a second solution comprising the compound of formula (A). Preferably, the base is n-butyl lithium. Preferably, the catalyst is an alkali or alkaline earth metal halide. Preferably, the solvent is tetrahydrofuran. Preferably, the addition of the first solution is at about -110 to about -50°C. Preferably, the addition of the second solution is at about -110 to about -50°C. Preferably, the addition of the second solution takes about 5 to about 180 minutes

The reaction can be carried out at a temperature range of -110 to 0°C. However, applicant has found that if the temperatures rises above about -50°C, by-products may be produced; and further if the temperature of the reaction is below about -110°C, the reaction rate slows down. Therefore, the optimal temperature range of the reaction is -110° to -50°C.

The present invention relates to processes for the production of chirally pure rosuvastatin intermediates, which can be used for the preparation ofHMG-CoA reductase inhibitors, said process comprising of the following steps of:
The present invention is directed towards a process of preparation of the compound of Formula (I) and formula (II), said process comprising of the steps of:
   a) Reacting a suitable salt with the chiral base of the compound of the formula (IX) to obtain the compound of the formula (X);
   b) treating the compound of formula (X) with an acid, to obtain the compound of the formula (XI),
      reacting the compound of formula (XI) of step (b) with pivaloyl chloride in the presence of base and in a reaction temperature of -50°C to 0°C, to obtain the compound of the formula (XII). The compound of formula (XII) is a useful intermediate for the preparation of compound of formula (I) and (II).

In any of the embodiments of the above processes, and compounds, the group R₁ is preferably C1-C4 group, more preferably methyl; the group R₂ is preferably C1-C4 group, more preferably t-butyl, the group R₈ or R₉ is preferably a C₆ to C₁₂ aryl, C₁₋₁₅ alkyl, more preferably R8 is methyl and R9 is phenyl, the the group Z is preferably a silyl group, more preferably a tert-butyldimethylsilyl.

The compounds of the formula (I) and (II) prepared by the process of the invention may be used to prepare statins for treatment of hyperlipidemia. Statins can be combined with a pharmaceutically acceptable excipient to prepare pharmaceutical compositions.

The statins that can be prepared include the following: 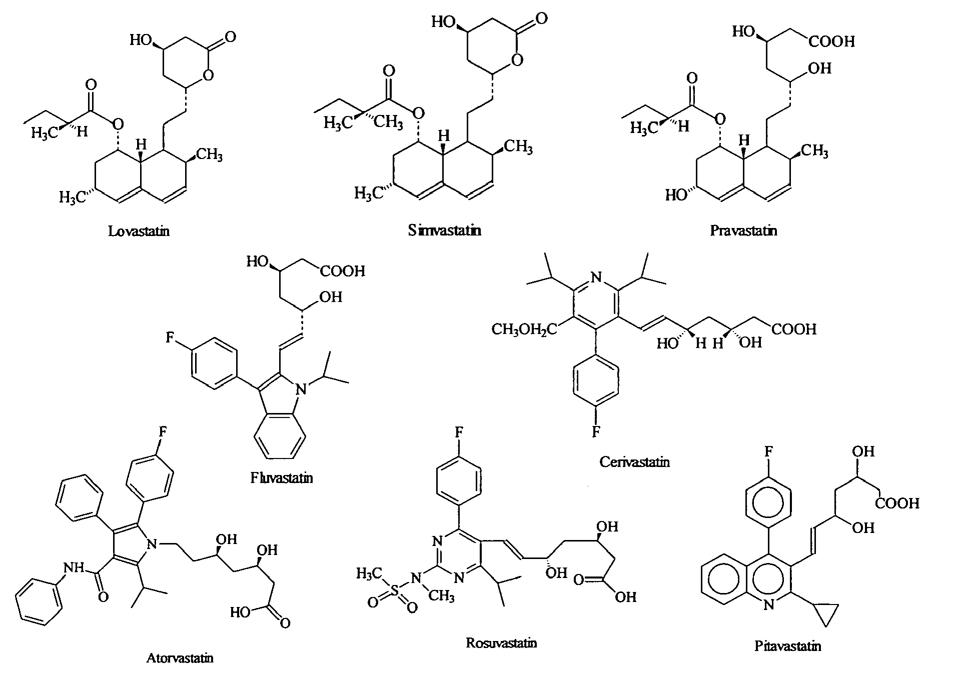 These statins can be prepared by reacting a ketone or aldehyde intermediate having the backbone of the particular statin with compounds of Formula I or II. The reaction is a Wittig reaction well known to one of ordinary skill of art. For example, Helvetica Chemica Acta, vol. 90 (2007), which is incorporated herein by reference, discloses a pitavastatin aldehyde precursor having the structure: 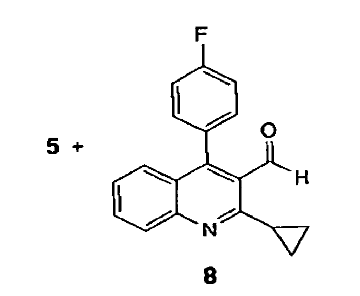 which is reacted with a silyl protected phosphonate. Preferably the statin is rosuvastatin.

WO2007/041666 and WO2006/091771, incorporated herein by reference, further disclose preparation of rosuvastatin through the Wittig reaction. After the Wittig reaction, the protecting group (Z) is removed, followed by reduction to obtain a diol, followed by hydrolysis of the ester to obtain a pharmaceutically acceptable salt.

Having described the invention with reference to certain preferred embodiments, other embodiments will become apparent to one skilled in the art from consideration of the specification. The invention is further defined by reference to the following examples describing in detail the process and compositions of the invention. It will be apparent to those skilled in the art that many modifications, both to materials and methods, may be practiced without departing from the scope of the invention.

### NMR data for compound of formula X-TBDMS

0.12 (d,6H), 0.88 (s,9H); 1.46 (s,9H); 1.62 (d,3H); 2.33 (dd,1H); 2.36 (m,2H); 2.48 (q, 1 H) ; 2.56 (dd, 1H); 4.41 (q, H); 7.38 (m, 1H); 7.42 (m, 4H)

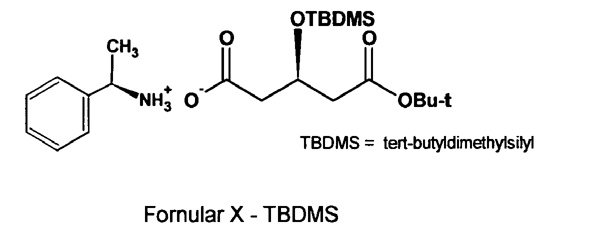

### Chromatography Data

### For Rosuvastatin Ca

BUFFER: -0.05% v/v Acetic acid glasial pH 3.5 with 5% Ammonium hydroxide.
ELUENT (A): - Mix.60% buffer 35% acetonitrile 5% ethanol.
ELUENT (B): -55% buffer 45% ethanol.
ELUENT (C): - ETHANOL.
COLUMN: -Discovery HS C18,3µm(150x4.6)mm
FLOW: -0.5ml/min, INJ.VOL.: -10µl,WAVELENGTH: -243nm.
COLUMN TEMP.: - 20°C,AUTOSAMPLER TEMP.: -4°C
RUN TIME: -25.0 MIN, EQUILIBRATION TIME: -7.0 MIN GRADIENT:-

### Gradient:

| | TIME | FLOW | %A | %B | %C | %D | CURVE |
|---|---|---|---|---|---|---|---|
| | 0.00 | 0.5 | 100 | 0 | 0 | 0 | 6 |
| | 15.00 | 0.5 | 0 | 100 | 0 | 0 | 6 |
| | 20.00 | 0.5 | 0 | 93 | 7 | 0 | 6 |
| | 25.00 | 0.5 | 0 | 64 | 36 | 0 | 6 |
| | 25.10 | 0.5 | 100 | 0 | 0 | 0 | 6 |

### Instrument

Gas Chromatograph equipped with Flame Ionisation Detector.

### Column

DB 17, 30 m x 0.53 mm x 1.0 µm film thickness, Agilent C/N: 125-1732 or equivalent.

### Chromatographic Conditions

| | | |
|---|---|---|
| a.) | Initial oven temperature | : 40°C |
| b.) | Initial hold time | : 3.0 minute |
| c.) | Initial-1 ramp rate | : 20°C / minute |
| d.) | Intermediate-1 oven temperature | : 160°C |
| e.) | Intermediate-1 hold time | : 10.0 minute |
| f.) | Initial-2 ramp rate | :10°C / minute |
| g.) | Intermediate-2 oven temperature | : 210°C |
| h.) | Intermediate-2 hold time | : 10.0 minute |
| i.) | Final ramp rate | : 20°C / minute |
| j.) | Final oven temperature | : 270°C |
| k.) | Final hold time | : 10.0 minute |
| l.) | Injector temperature | : 180°C |
| m.) | Detector temperature | : 300°C |
| n.) | Carrier gas (He) flow | : 10.0ml/min |
| o.) | Mode | : Constant flow |
| p.) | Injection volume | : 1.0 µl |
| q.) | Split ratio | : Splitless |

Temperature and flow rate may be varied in order to achieve the required system suitability.

### Diluent

Acetonitrile

### Preparation of system suitability solution

Weighed accurately about 20mg of each: TBDMS-OH, DMMP, MBSG and 19TBPO into 10ml volumetric flask, dissolve and brought to volume with diluent. Transferred 1ml of the stock solution into 10ml volumetric flask and brought to volume with diluent.

### System suitability test

Injected system suitability solution.

Typical retention times are about 4minutes for TNDMS-OH peak, about 6.5 minutes for the DMMP peak, About 14.5minutes for the MBSG peak and 31.5minutes for the 19TBPO peak.

### Preparation of sample solution

Weighed accurately 20mg of sample into 10ml volumetric flask, dissolved and brought to volume with diluent.

### EXAMPLES

### Example 1

### Preparation of 3-hydroxy dihydrogen glutarate (TBDMS protected) (protected diacid) from 3-hydroxy diethyl glutarate (TBDMS protected) through hydrolysis (Formula V from hydrolysis of Formula IV)

To a 5-liter four neck round bottom flask under nitrogen atmosphere added 163 g 3-hydroxy diethyl glutarate (TBDMS protected) and 815 ml methanol at 25 to 30°C, and added 45.10 g sodium hydroxide with 326 ml water to the reaction mass over the period of 1 hr at 30to 32°C. Stirred reaction mass at 30 to 32°C. Starting material was not detected after 24 hr by TLC. White colored disodium salt was observed. Added 500 g 10% hydrochloric acid solution to adjust pH of reaction mass to 3.5 to produce a clear solution. Added 275 g sodium chloride to saturate reaction mass and extracted product in 4x 650ml dichloromethane, evaporated solvent under vacuum at 40 to 50 °C to get white colored crude product which was further stirred with 600 ml hexane and filtered. Dry the product under vacuum at 50°C to get 109 g pure product.

Yield 81.55 %, purity 94.2% (Area % by GC)

### Example 2

### Preparation of 3-hydroxy glutaric anhydride (TBDMS protected) from 3-hydroxy dihydrogen glutarate (TBDMS protected) (Formula VI from Formula V)

To a 2-liter four neck round bottom flask under nitrogen atmosphere added 180 g solid 3-hydroxy dihydrogen glutarate (TBDMS protected) and 630 ml acetic anhydride at 25 to 30°C and reflux reaction mass for 2 hr at 130-135 °C. Monitored by TLC and GC, starting material was not found in 2.0 hr. Acetic anhydride was distilled under vacuum at 75-80°C completely and crude product was crystallized through 180 ml hexane and dried pure brown colored product under vacuum at 40°C. Obtained 171 g pure product.

Yield 100% purity 99.5%(Area % by GC)

### Example 3

### Preparation of racemic methyl hydrogen 3-hydroxy glutarate (TBDMS protected) from 3-hydroxy glutaric anhydride (TBDMS protected) (Formula VII from Formula VI)

To a 2-liter four neck round bottom flask under nitrogen atmosphere added 20 g 3-hydroxy glutaric anhydride (TBDMS protected), 200 ml methanol and reflux reaction mass for 12 hr at 60 to 65°C. Monitored reaction progress by GC, starting material was not detected. Allowed reaction mass temperature up to room temperature ("RT") and added 2-3 drop of hydrochloric acid and 100 ml water. Added sodium chloride to saturate reaction mass at room temperature. Extracted product with 200 ml dichloromethane. Aqueous layer was extracted with 20ml dichloromethane and combined organic layer was dried over sodium sulphate. Solvent was evaporated to give 20.0 g product.

Yield 88.4% purity 94.5% (Area % by GC)

### Example 4

### Preparation of racemic methyl tert-butyl 3-hydroxy glutarate (TBDMS protected ) from methyl hydrogen 3-hydroxy glutarate (TBDMS protected) through esterification (Formula VIII from esterification of Formula VII)

To a 100ml four neck round bottom flask under nitrogen atmosphere added 5.0 g methyl hydrogen 3-hydroxy glutarate (TBDMS protected), 35 ml dichloromethane at RT and cooled reaction mass to 0°C, added 4.1 g dicyclohexylcarbodimide (DCC), 15 ml tert-butanol, 5.5 g dimethylaminopyridine. Stirred reaction mass for 40 hr at room temperature. Reaction progress was monitored by GC, starting material was not detected after 40 hr.

Filtered white solid that is DHU (urea). Solvent was evaporated from mother liquor and dissolved in 50 ml toluene. Toluene layer was washed with 3 × 25 ml 10% hydrochloric acid to remove excess dimethylaminopyridene at room temperature. Organic layer was washed with 50 ml brine solution followed by drying by sodium sulphate. Solvent was evaporated up to complete dryness to give title compound 4.3 g.

Yield 71.31% purity 88.63% (Area % by GC)

### Example 5

### Preparation of racemic tert-butyl hydrogen 3-hydroxy glutarate (TBDMS protected from racemic methyl tert-butyl 3-hydroxy glutarate (TBDMS protected) (hydrolysis of Formula VIII)

To a 2.0-liter four necks round bottom flask under nitrogen atmosphere added 100 g racemic methyl tert-butyl 3-hydroxy glutarate (TBDMS protected) and 500 ml absolute ethanol at 25 to 30°C. Reaction mass temperature was raised up to 48-52°C. Added 332 ml 1N sodium hydroxide solution in four lots at 48-52°C over the period of 3.0 hr. Lot wise additions was preferred to avoid diacid formation. Stirred reaction mass for 7-8 hr at the same temperature. Starting material was not detected by GC monitoring after 7 hr. Evaporated absolute ethanol under vacuum at 40-45°C. Added 400 ml water and 50ml absolute ethanol to reaction mass at RT. Aqueous layer containing product was washed with 2 × 300 ml hexane at RT. Added toluene 300ml to the aqueous layer and adjust pH to approx 5-6 using 25 ml hydrochloric acid, the layers were separated and the aqueous layer was extracted with 2 × 300 ml toluene. Combined organic layers were washed with brine solution and dried over sodium sulphate. Evaporated organic layer under vacuum at 50-55°C completely to give desired product 85.5 g.

Yield 92.93% Purity 98.5% (Area % by GC)

### Example 6

### Preparation of racemic tert-butyl hydrogen 3-hydroxy glutarate (TBDMS protected) from 3-hydroxy glutaric anhydride (TBDMS protected) (Formula IX from ring opening of Formula VI)

To a 250ml four neck round bottom flask under nitrogen atmosphere added 10 g 3-hydroxy glutaric anhydride (TBDMS protected),100 ml tert-butanol at RT. Cooled reaction mass to 20 to 22°C. Added 20 g dimethylaminopyridine over a period of 2 min. Stirred reaction mass at 30-32°C. Starting material was not detected after 20 hr by GC and TLC monitoring. Evaporated solvent from reaction mass under vacuum at 45-50°C to get solid residue. Residue dissolved 100 ml toluene and washed organic layer with 2 × 50 ml hydrochloric acid at RT. Aqueous layer was extracted with 2 × 50ml toluene. Combined toluene layer was washed with 50 ml water and 50ml brine solution. Toluene layer was concentrated to dryness to give 7.5 g title compound as oil.

Yield 57.69% purity 87.78% (Area % by GC)

### Example 7

### Preparation of tert-butyl hydrogen 3(R )-hydroxy glutarate (TBDMS protected) from racemic tert-butyl hydrogen 3-hydroxy glutarate (TBDMS protected) (mono acid) (Formula XI from Formula IX)

### First chiral purification:

To a four necks round bottom flask under nitrogen atmosphere added 5.0 g racemic tert-butyl hydrogen 3-hydroxy glutarate (TBDMS protected) and 15 ml isopropyl alcohol at RT. Slowly added 2.1 g R(+) phenylethyl amine at 25 to 30°C over a period of 15 min by maintaining temperature bellow 30°C. Stirred reaction mass for 6.0 hr at 25 to 30°C. White colored salt was observed.

Raised temperature of reaction mass up to 65 to 70°C to get clear solution. Allowed temperature of reaction mass up to 20°C and stirred for 12.0 hr. Filtered crystallized solid at 20°C and dry under vacuum at 40-45°C to give 2.5 g pure salt. Mother liquor was subjected to recycling. Obtained 2.5 g phenylethyl amine salt of mono acid was added in fresh 100 ml four neck round bottom flask, added 25 ml water, 10 g sodium chloride, and 1.3 equivalent 10% hydrochloric acid, stirred reaction mass for 2.0 hr and extracted product in 3 × 10 ml dichloromethane at room temperature. Solvent was evaporated up to dryness to give 1.77 g product.

Yield 35.40%, Purity 98.5% by GC, ee by HPLC 85%

### Second chiral purification:

Obtained 1.77 g oil was dissolved in 5.0 vol isopropyl alcohol at RT and slowly added 0.74 g R(+) phenyl ethyl amine over a period of 5 min and stirred reaction mass for 6.0 hr at 20 to 25°C. White colored salt formation was observed. Heated reaction mass up to 65 to 70°C to get clear solution. Allowed temperature of reaction mass up to 20°C and stirred for 12.0 hr. Filtered crystallized solid at 20°C and dry under vacuum at 40-45°C to give 1.94 g pure salt. Mother liquor was subjected to recycling. Obtained 2.5 g phenylethyl amine salt of mono acid was added in fresh 100 ml four neck round bottom flask, added 19.4 ml water, 5.82 g sodium chloride, and 1.3 equivalent 10% hydrochloric acid, stirred reaction mass for 2.0 hr and extracted product in 3 × 7 ml dichloromethane at RT. Solvent was evaporated up to dryness to give 1.38 g pure product.

Yield 78% purity 99.5%( by GC % area), ee purity by HPLC 99.55%

### Example 8

### Determination of enantiomeric excess:

### Derivatization of tert-butyl hydrogen 3-hydroxy glutarate (TBDMS protected) (mono acid) by 4-bromo benzyl bromide. (Formula XI derivatized)

To a 50 ml round bottom flask under nitrogen atmosphere added 1.0 g tert-butyl hydrogen 3-hydroxy glutarate (TBDMS protected) (mono acid ),10 ml dimethyl formamide, 0.86 g 4-bromo benzyl bromide, 0.65 g potassium carbonate at 25to 30°C. Stirred reaction mass for 6.0 hr and monitored reaction progress, starting material was not detected by TLC. Extracted product in 15 ml dichloromethane and organic layer was washed with 3 × 10 ml water. Solvent was evaporated up to dryness and obtained 1.1 g product which was subjected to determination of enantiomeric excess by chiral HPLC.

Yield 78.57% Enantiomeric excess purity 99.55%

### Example 9

### Recycling

Mother liquor obtained from example 7 during first chiral purification and second chiral purification which contain mixture of compound XI and tert-butyl hydrogen 3(S)-hydroxyglutarate (TBDMS protected) R(+) phenylethyl amine salts, was placed in four neck RBF and added 10% hydrochloric acid to adjust pH 5 to 5.5, extracted product in 5.0 vol toluene and concentrated up to dryness to get mono acid oil. Obtained product was taken in fresh four neck RBF under nitrogen atm and added 5.0 vol absolute ethanol and heated reaction mass up to 50°C, added 1.2 equivalent 1N sodium hydroxide solution and stirred. After completion of reaction adjust pH of reaction mass up to 3.5-4.0 to get clear solution. Added sodium chloride to saturate reaction mass, extracted product in dichloromethane and solvent was evaporated to dryness to get solid diacid i.e. 3-hydroxy dihydrogen glutarate (TBDMS protected) which was subjected to formation of cyclic anhydride and further conversion in monoacid i.e. tert-butyl hydrogen (3R) hydroxy glutarate (TBDMS protected) by using same procedure of examples 3 to 8.

### Example 10

### Preparation of tert-butyl hydrogen 3(R)-hydroxy glutarate(TBDMS protected) from methyl tert-butyl 3(R)-hydroxy glutarate (TBDMS protected) (Formula XI from methyl ester of Formula XI (Formula A)

To a 2.0 liter four neck round bottom flask under nitrogen atmosphere added 100 g methyl tert-butyl 3(R)-hydroxy glutarate (TBDMS protected) and 500 ml absolute ethanol at room 25 to 30°C. Reaction mass temperature was raised up to 48-52°C. Added 332 ml 1N sodium hydroxide solution in four lots at 48-52°C over the period of 3.0 hr. Lot wise addition was preferred to avoid diacid formation. The.reaction mass was stirred for 7-8 hr at same temperature. Starting material was not detected by GC monitoring after 7hr. Evaporated absolute ethanol under vacuum at 40-45°C. Added 400 ml water and 50ml absolute ethanol to reaction mass at RT. Aqueous layer containing product was washed with 2 × 300 ml hexane at RT. Added toluene 300 ml to the aqueous layer and adjust pH up to approx 5-6 using 25 ml hydrochloric acid, layer were separated and aqueous layer was extracted with 2 × 300 ml toluene. Combined organic layers were washed with a brine solution and dried over sodium sulphate. Evaporated organic layer under vacuum at 50-55°C completely to give desired product 85.5 g.

Yield 92.93% Purity 98.5% (Area % by GC)

### Example 11

### Preparation of mixed anhydride of tert-butyl hydrogen 3-(R) hydroxy glutarate(TBDMS protected) (Formula XII from Formula XI)

To a four neck round bottom flask under nitrogen atmosphere was added 3.84 g methylchloroformate and 180 ml toluene at RT. The reaction mass was cooled to -40°C. Added was a mixture of 10 g tert-butyl hydrogen 3(R)-hydroxy glutarate (TBDMS protected), 20 ml toluene, 5.39 g triethylamine over a period of 40 min. The temperature was slowly raised to 0°C within 1.5 hr. The reaction was quenched with 100 ml water at 0°C. The layers were separated immediately at 0°C. The organic layer was washed with 1×100 ml water, 2x50 ml sat. sodium bicarbonate solution followed by 50 ml of brine solution. The washed organic layer was concentrated under vacuum at 45-50°C to remove completely the toluene to give the mixed anhydride 12.2 gm. The product was used in the next step without any further purification.

Yield 100 % purity 96.75 %( by GC % Area)

### Example 12

### Preparation of t-butyl (3R)-3-(t-butyl dimethylsilyloxy)-6dimethoxyphosphinyl-5-oxohexanoate(19TBPO) from mixed anhydride (Formula I from Formula XII)

To a four neck round bottom flask under nitrogen atmosphere was added 38.5 g dimethylmethylphosphonate and 377.6 ml tetrahydrofuran at RT. The reaction mass was cooled to -78°C. To the reaction mass was added 240 ml 1.6 M n-butyl lithium solution in hexane over a period of 1.0 hr at -78 to -80°C. The reaction mass was stirred for 1.5 hr at - 78 to -80°C for to allow for anion formation. 47.2 g mix anhydride in 377.6 ml tetrahydrofuran was added at -78 to -80°C over a period of 45 min. After completion of the addition, the reaction mass was stirred for 2 min and 200 ml 20% ammonium chloride solution was added at -70 to -80°C. The reaction mass was raised to RT and the pH adjusted to 5 to 5.5 using 200 ml 10% hydrochloric acid. 236 ml toluene was added with stirring. The layers were separated and the organic layer was washed with 211 ml 1N hydrochloric acid followed by 211 ml brine solution, The organic layer was concentrated to dryness to give a crude pale brown colored transparent product 52.0 g.

### Purification of crude 19TBPO (Formula I)

Obtained 52.0 g crude product was dissolved in 500 ml toluene. Toluene layer was washed with 2 × 350 ml 2% sodium carbonate solution and 350 ml water. Aqueous layer was extracted with 2 × 250 ml toluene. Combined organic layer was washed with 500 ml brine solution. Organic layer was concentrated up to dryness to give 40 g of pure product. The 40 g product was dissolved in 200 ml acetonitrile and washed with 3 x 200 ml hexane at RT. Evaporated acetonitrile layer under vacuum at 50°C to give 27.84 g pure product.

Yield 52.42 %, Purity by GC 93.47% (by GC % area)

### Example 13

### Preparation of 3(R)-3(tert-butyldimethylsilyloxy)-5-oxo-6-triphenyl-phoporanylidene hexanoate from mixed anhydride (Formula II from Formula XII)

To a four neck round bottom flask under nitrogen atmosphere added 32.93 g methyltriphenylphosphonium bromide and 88ml tetrahydrofuran at RT. The reaction mass was cooled to -50-55°C and 57.5 ml 1.6 M n-butyl lithium solution in hexane was added over a period of 0.5 hr at -50-55°C. The reaction temperature was raised to 0°C over a period of 1.5 hr and again cooled to -70°C. 18.0 g of mixed anhydride with 52.86 ml toluene was added at -55 to -65°C over a period of 2.0 hr. After completion of the addition, the reaction was raised slowly to 0°C over a period of 1.0 hr. 36.0 ml water was added over a period of 15 min at 0°C. The organic layer was separated and the aqueous layer extracted with 18.0 ml toluene. The combined organic layers were washed at RT with 2 × 18 ml sat. sodium bicarbonate solution, 18.0 ml brine solution. The organic layer was dried over magnesium sulphate and concentrated to dryness to give a pale brown colored transparent product 22.44 g . Obtained product was used in the next step without further purification.

Yield 84.0%

### Example 14

### Preparation of t-butyl (3R)-3-(t-butyl dimethylsilyloxy)-6-dimethoxyphosphinyl-5-oxohexanoate(19TBPO) from t-butyl methyl (3R)-3-(t-butyl dimethylsilyloxy)-glutarate (MBSG, Formula XIII) (Formula I from Formula A)

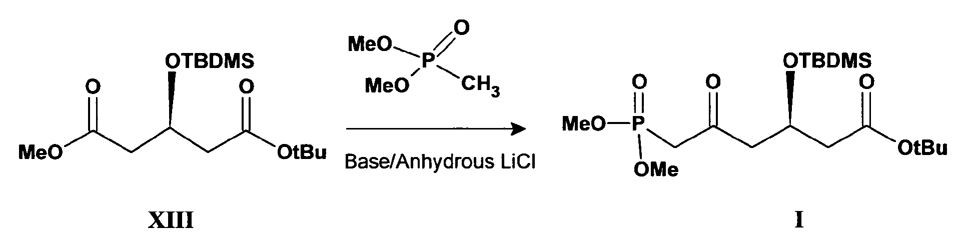

To a four neck round bottom flask under nitrogen atmosphere was added 7.44 g dimethyl methylphosponate, 50 ml tetrahydrofuran, and 1.91 g anhydrous lithium chloride at RT. The reaction mass was cooled to -78 to -80°C and 29 ml of 1.6 M n-butyl lithium solution in hexane was added over a period of 30 min at -78 to -80°C. The reaction mass was stirred for 2 hr at -78 to -80°C to allow anion formation. 5 g of formula (XIII) with 50 ml tetrahydrofuran was added at-78 to -80°C over a period of 120 to 135 min. After the addition was complete, the reaction mass was stirred for 45-60 min followed by addition of 15 ml 20% ammonium chloride solution at -65 to -78°C. The reaction mass temperature was raised to RT and the pH adjusted to 1-2 using 25 ml 20% hydrochloric acid. 25 ml Toluene was added and the mixture stirred for 60 min. The layers were separated and the organic layer washed with 50 ml saturated sodium chloride solution. The organic layer was concentrated under vacuum up to dryness to give a pale yellow colored transparent product 5.5 g, assay 77.96%, purity 71.5% (by GC % Area) and yield 67.52%.

Example 15-Preparation of Compound 20TB by Wittig reaction from 19TBPH (Example 4 of WO2007/041666)

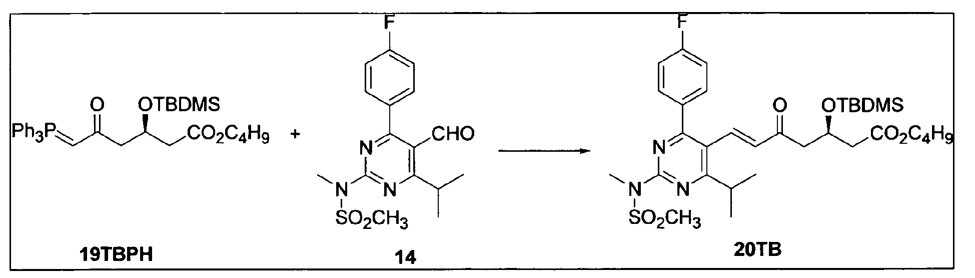

A 100 ml flask, protected from light and provided with N₂ flow was charged with Compound 14 (3.6 g, 10.5 mmol), Compound 19TBPH (9.05 g, 15.7 mmol), and dry toluene (36 ml, 10 vol relative to Compound 14). The reaction mixture was heated to about 100°C for 19.5 hrs. A sample of the reaction mixture was analyzed by HPLC, and contained 1.7% of Compound 14.

Anhydrous MgCl₂ (2 g, 2 equivalents relative to Compound 19TBPH) was added to the reaction mixture and the reaction mixture was stirred at 100°C for 2 hrs. The reaction mixture was cooled to 0°C for 2 hours, and filtered without washing the solid. A filtrate was obtained and was washed twice with H₂O (100 ml each) and the solvent was evaporated, yielding 7.56 g of a brown solid.

Example 16: Preparation of Compound 20M by Wittig Reaction (Example 5 of WO2007/041666)

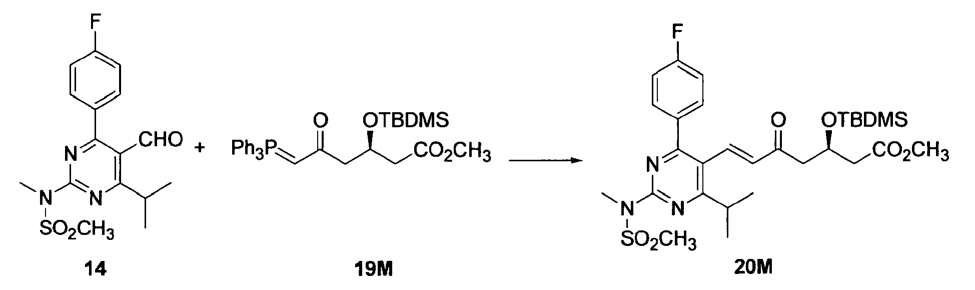

A 250 ml flask, protected from light and provided with N₂ flow was charged with Compound-14 (4.38 g, 12.5 mmol), Compound 19M (10 g, 18.7 mmol), and extra dry toluene (100 ml). The reaction mixture was heated to about 100°C for 15 hrs. After the completion of the reaction, anhydrous MgCl₂ (4.8 g, 2.7 eq.) was added to the reaction mixture and the reaction mixture was heated for 2 hours at about 100°C. The reaction mixture was cooled to 0°C over a period of about 2 hours, filtered, and washed with 45ml of toluene, yielding 12.73g of a viscous oil.

**Example 17: Preparation of Compound 21TB in HCl/THF** (Example 7 of WO2007/041666)

A mixture of HCl (32% in water, 0.57 g), water (2 mL), and THF (17.5 mL) was prepared. 5.4 mL of this mixture were added dropwise to a solution of Compound 20TB (2.7g) in THF (8.1 mL). The reaction mixture was stirred at ambient temperature overnight, until monitoring of the reaction by TLC indicated completion of the reaction.

Ethyl acetate (20 mL) was added to the reaction mixture and the reaction mixture was washed with water (20 mL). An aqueous layer formed, and was extracted with ethyl acetate (20 mL). The organic layers were combined and washed with an aqueous solution of Et₃N (2 x 5 mL) at a pH of about 10.5. The organic layer was dried over MgSO₄ and the solvent was removed under reduced pressure, yielding an oil of Compound 21TB (2.03 g).

**Example 18: Preparation of Compound 22TB (TBRE)** (Example 16 of WO2007/041666)

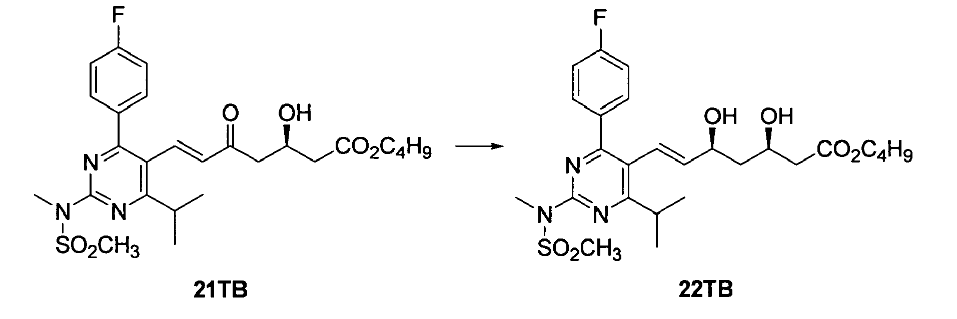

To a solution of 2 1 TB (1 g) in dry THF (26 mL) and dry methanol (7 mL), a solution of diethylmethoxyborane (1M) in THF (2 mL) was added at about -78°C, forming a reaction mixture. The reaction mixture was stirred for 0.5 hour, NaBH₄ was added, and the stirring was continued for 3 hours. Acetic acid (1.2 mL) was added to the reaction mixture and the reaction mixture was warmed to ambient temperature.

Ethyl acetate (150 mL) was added to the reaction mixture and the pH was adjusted to 8 by addition of concentrated NaHCO₃ water solution. The layers were separated, and water was extracted by adding an additional amount of ethyl acetate (50 mL). The organic layers were combined and dried over MgSO₄. The solvents were then evaporated under reduced pressure, leaving a residue. The residue was treated with methanol and then the methanol was evaporated. Methanol treatment and evaporation was performed two more times, yielding crude Compound 22TB (TBRE) (0.87 g, 86%).

**Example 19: Conversion of Compound 22TB into rosuvastatin Ca with extraction in ethyl acetate** (Example 17 of W02007/041666).

A 1 L reactor equipped with a mechanical stirrer was charged with EtOH (3 L), water (1800 mL), and TBRE (600 g), forming a reaction mixture. NaOH (47%, 1.2 eq, 114 g) was slowly added to the reaction mixture, at RT. The reaction mixture was stirred at about RT for two hours. The reaction mixture was filtered under reduced pressure with Synter and Hyflo to eliminate the small particles present. The reaction mixture was concentrated under reduced pressure at about 40°C until half the volume of the reaction mixture remained.

Water (2000 mL) was added to the reaction mixture and the reaction mixture was stirred at about RT for 5 minutes. An aqueous phase and an organic phase formed. The phases were separated, and the aqueous phase was washed with ethyl acetate (3000 mL) and stirred at RT for half an hour. The organic phase was discarded.
The aqueous phase was concentrated under reduced pressure at about 40°C until half the volume remained. Water (2800 mL) was added to the aqueous phase and the aqueous phase was stirred at about RT for 5 minutes. CaCl₂ (124 g) was added to the aqueous phase in portions over a period of about 10 minutes at a temperature of about RT. The aqueous phase was then stirred at about RT for about 1 hour, filtered, and washed with 1200 mL of water, yielding a powdery compound (491 g, 88%).
Further aspects and features of the invention are defined in the following numbered clauses:
1. A salt of a base cation having the following formula (X): 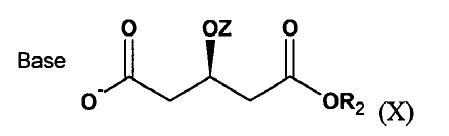 wherein the base is a chiral base;
   wherein Z is a hydroxy protecting group and R₂ is an alkyl group of 1-5 carbon atoms.
2. The salt of clause 1, wherein the chiral base that is an amine.
3. The salt of clause 1 or 2, wherein the salt has the following structure: 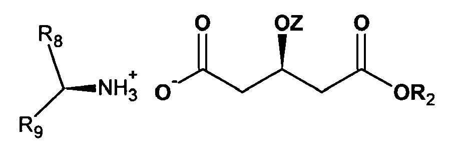 wherein R₈ and R₉ are different and each is independently selected from the group consisting of C₁₋₁₅ alkyl, and C₆ to C₁₂ aryl.
4. The salt of any of the preceding clauses, wherein one of R₈ or R₉ is a C₆ to C₁₂ aryl, C₁₋₁₅ alkyl,
5. The salt of any of the preceding clauses, wherein one of R₈ or R₉ is a C₁-C₄ alkyl.
6. The salt of any of the preceding clauses, wherein R₈ is methyl and R₉ is phenyl.
7. The salt of any of the preceding clauses, wherein the amine is 1-phenylethylamine, 1-phenylpropylamine, 1-phenylisobutylamine, 1-phenylbutylamine and 1-phenylpentylamine.
8. The salt of any of the preceding clauses, wherein the amine is R (+)-phenylethylamine.
9. The salt of any of the preceding clauses, wherein the amine is S (-)-phenylethylamine.
10. The salt of any of the preceding clauses, wherein the alkyl group is substituted with hydroxy, carboxyl, C₁-C₄ alkyl, C₆-C₁₂ alkoxy, C₆-C₁₂ aryl, C₆-C₁₂ arylalkyl, C₆-C₁₂ cycloalkyl and amino.
11. The salt of clause 1, wherein R₂ is a C₁-C₄ group.
12. The salt of clause 1, wherein R₂ is a t-butyl group.
13. The salt of clause 1, wherein Z is a silyl group.
14. The salt of clause 1, wherein Z is a trialkylsilysl.
15. The salt of clause 1, wherein Z is triethylsilyl, triisopropylsilyl, tert-butyldiphenylsilyl or tert-butyldimethylsilyl.
16. The salt of clause 1, wherein Z is tert-butyldimethylsilyl.
17. A process for preparing a compound of formula X comprising:
   a) reacting a compound of formula IX having the structure: 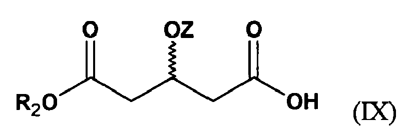 with a chiral base to obtain a salt of the following structure: 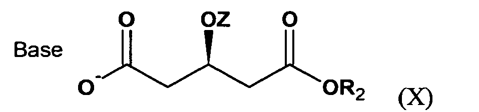
   wherein Z is a hydroxy protecting group and R₂ is an alkyl group of 1-5 carbon atoms
18. The process of clause 17, wherein the chiral base that is an amine.
19. The process of clause 17-18, wherein the salt has the following structure: 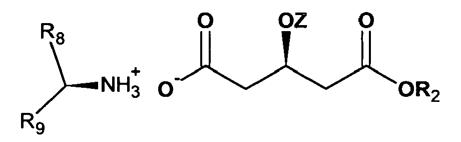 Wherein R₈ and R₉ are different, and each of R₈ and R₉ is independently selected from the group consisting of C₁₋₁₅ alkyl, and C₆ to C₁₂ aryl.
20. The process of clause 19, wherein one of R₈ or R₉ is a C₁-C₄ alkyl.
21. The process of clause 19, wherein R8 is methyl and R₉ is phenyl.
22. The process of any of the preceding clauses, wherein the amine is 1-phenylethylamine, 1-phenylpropylamine, 1-phenylisobutylamine, 1-phenylbutylamine and 1-phenylpentylamine.
23. The process of clause 18, wherein the amine is R (+)-phenylethylamine.
24. The process of clause 18, wherein the amine is S (-)-phenylethylamine.
25. The process of any of the preceding clauses, wherein the alkyl group is substituted with hydroxy, carboxyl, C₁-C₄ alkyl, C₆-C₁₂ alkoxy, C₆-C₁₂ aryl, C₆-C₁₂ arylalkyl, C₆-C₁₂ cycloalkyl and amino.
26. The process of any of the preceding clauses, wherein R₂ is a C₁-C₄ group.
27. The process of any of the preceding clauses, wherein R₂ is a t-butyl group.
28. The process of any of the preceding clauses, wherein Z is a silyl group.
29. The process of any of the preceding clauses, wherein Z is a trialkylsilysl.
30. The process of any of the preceding clauses, wherein Z is triethylsilyl, triisopropylsilyl, tert-butyldiphenylsilyl or tert-butyldimethylsilyl.
31. The process of any of the preceding clauses, wherein Z is tert-butyldimethylsilyl.
32. The process of any of the preceding clauses, wherein the chiral base is in the amount of 1-2 molar equivalents based on the compound of formula (IX).
33. The process of any of the preceding clauses, wherein the solvent is selected from a group of C₁- C₄ aliphatic alcoholic solvents, C₆- C₁₀ aromatic and aliphatic hydrocarbons, C₂-C₈ aliphatic esters, C₄-C₈ ethers, C₁-C₆ chlorine substituted alkyl.
34. The process of any of the preceding clauses, wherein the solvent is selected from the group consisting of methanol, ethanol, isopropyl alcohol, n-butanol, t-butyl alcohol, isopropyl alcohol, toluene, benzene, hexanes, cyclohexane, methyl acetate, ethylacetate, t-butylacetate, isopropyl acetate, methyl t-butyl ether, tetrahydrofuran, dichloromethane, dichloroethane, chloroform, carbon tetrachloride, tetrachloroethylene and tetrachloroethane.
35. The process of any of the preceding clauses, wherein the solvent is isopropanol.
36. The process of any of the preceding clauses, wherein the temperature is about -60°C to about 80°C.
37. The process of any of the preceding clauses, wherein the temperature is about 0°C to about 70°C.
38. A process for preparing a statin further comprising converting the compound obtained in any of the preceding clauses to a statin.
39. A process for preparing the compound of formula: 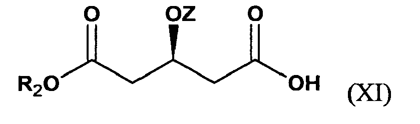 comprising reacting the salt of formula: 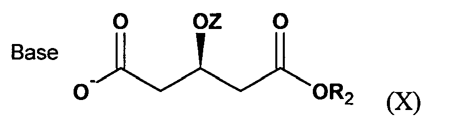 with an acid,
   wherein the base is a chiral base,
   wherein Z is a hydroxy protecting group and R₂ is an alkyl group of 1-5 carbon atoms.
40. The process of clause 39, wherein the chiral base that is an amine.
41. The process of clause 39, wherein the salt has the following structure: 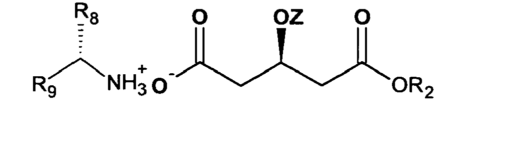 Or 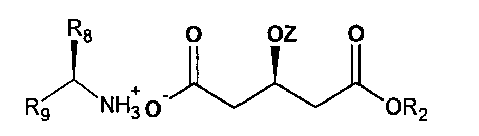 wherein R₈ and R₉ are different and each is independently selected from the group consisting of C₁₋₁₅ alkyl, and C₆ to C₁₂ aryl.
42. The process of clause 41, wherein one of R₈ or R₉ is a C₆ to C₁₂ aryl, C₁₋₁₅ alkyl,
43. The process of clause 41, wherein one of R₈ or R₉ is a C₁-C₄ alkyl,
44. The process of clause 41, wherein R₈ is methyl and R₉ is phenyl.
45. The process of clause 40, wherein the amine is 1-phenylethylamine, 1-phenylpropylamine, 1-phenylisobutylamine, 1-phenylbutylamine and 1-phenylpentylamine.
46. The process of clause 40, wherein the amine is R (+)-phenylethylamine.
47. The process of clause 40, wherein the amine is S (-)-phenylethylamine.
48. The process of clause 39, wherein the alkyl group is substituted with hydroxy, carboxyl, C₁-C₄ alkyl, C₆-C₁₂ alkoxy, C₆-C₁₂ aryl, C₆-C₁₂ arylalkyl, C₆-C₁₂ cycloalkyl and amino.
49. The process of clause 39, wherein R₂ is a C₁-C₄ group.
50. The process of any of the preceding clauses, wherein R₂ is a t-butyl group.
51. The process of clause 39, wherein Z is a silyl group.
52. The process of any of the preceding clauses, wherein Z is a trialkylsilysl.
53. The process of any of the preceding clauses, wherein Z is triethylsilyl, triisopropylsilyl, tert-butyldiphenylsilyl or tert-butyldimethylsilyl.
54. The process of any of the preceding clauses, wherein Z is tert-butyldimethylsilyl.
55. The process of clause 39, wherein the acid is a mineral acid or a C₁-C₈ organic acid.
56. The process of clause 39, wherein the acid is hydrochloric acid or acetic acid.
57. The process of clause 39, wherein the reaction is carried out in water.
58. The process of clause 39, wherein the acid is hydrochloric acid.
59. The process of any of the preceding clauses, wherein hydrochloric acid is used in a concentration range of 1N-10N.
60. The process of any of the preceding clauses, wherein the reaction temperature is about 0°C to 50°C.
61. A process for preparing a statin further comprising converting the compound obtained in any of the preceding clauses to a statin.
62. A process for preparing the compound of formula: 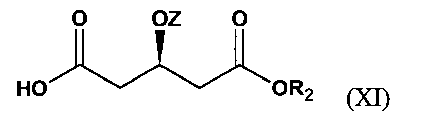
   a) reacting a compound of formula IX having the structure: 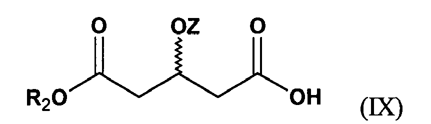 with a chiral base to obtain a salt of the following structure: 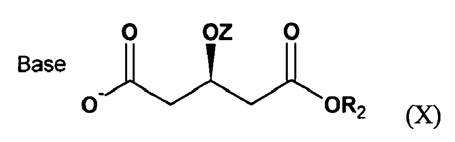 wherein the base is a chiral base;
      wherein Z is a hydroxy protecting group and R₂ is an alkyl group of 1-5 carbon atoms.
   b) reacting the salt with an acid.
63. A process for preparing a statin further comprising converting the compound obtained in clause 0 to a statin.
64. A salt having the following structure: 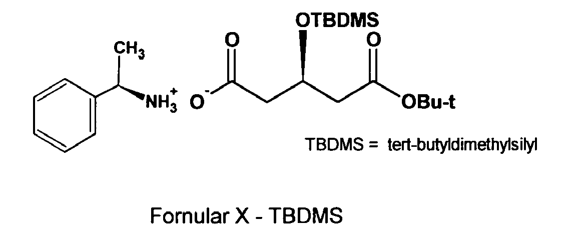
65. The salt of formula of clause 64 characterized by the following NMR data: 0.12 (d,6H), 0.88 (s,9H); 1.46 (s,9H); 1.62 (d,3H); 2.33 (dd,1H); 2.36 (m,2H); 2.48 (q, 1 H) ; 2.56 (dd, 1H); 4.41 (q, H); 7.38 (m, 1H); 7.42 (m, 4H).
66. The process of any of the preceding clauses, wherein the statin is preferably wherein the statin is lovastatin, simvastatin, pravastatin, fluvastatin, cerivastatin, atorvastatin, rosuvastatin, and pitavastatin, more preferably rosuvastatin, and preferably rosuvastatin.
67. Use of compounds of any of the preceding clauses in a process for the manufacture of a statin compound, preferably wherein the statin is lovastatin, simvastatin, pravastatin, fluvastatin, cerivastatin, atorvastatin, rosuvastatin, and pitavastatin, more preferably rosuvastatin.

## Claims

1. A process for the preparation of the compound formula (XII) comprising reacting the compound of formula (XI): 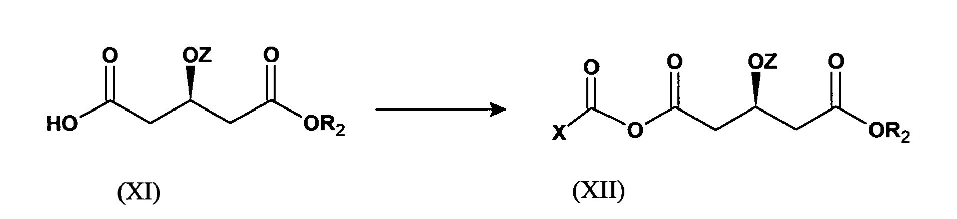 with a compound of formula R_{f}CO-Y in the presence of base, wherein Y is halogen, X is an alkoxy group of C₁ to C₅ carbon atoms or an alkyl group of C₁-C₅ carbons and R_{f} is a C₁-C₆ alkyl group.

2. The process of Claim 1, wherein R_{f} is a C₂-C₄ alkyl group.

3. The process of any of the preceding claims, wherein R_{f} is t-butyl, and Y is Cl, and wherein the compound of formula R_{f}CO-Y is pivaloyl chloride.

4. The process of any of the preceding claims, wherein the base is an organic amine, preferably wherein the organic amine is a trialkylamine of formula NRₐR_{b}R_{c} wherein Rₐ, R_{b}, and R_{c} are independently selected from C₁₋₆ alkyl groups.

5. The process of any of the preceding claims, wherein the reaction is carried out at a temperature range of -50°C to 50°C.

6. The process of any of the preceding claims, wherein R₂ is a C₁-C₄ group, and preferably wherein R₂ is a t-butyl group.

7. The process of any of the preceding claims, wherein Z is a silyl group, preferably wherein Z is a trialkylsilyl, more preferably wherein Z is triethylsilyl, triisopropylsilyl, tert-butyldiphenylsilyl or tert-butyldimethylsilyl, and most preferably wherein Z is tert-butyldimethylsilyl.

8. A process for preparing a statin further comprising converting the compound obtained in any of the preceding claims to a statin.

9. A process for preparing the compound of formula (I) from compound of formula (XII): 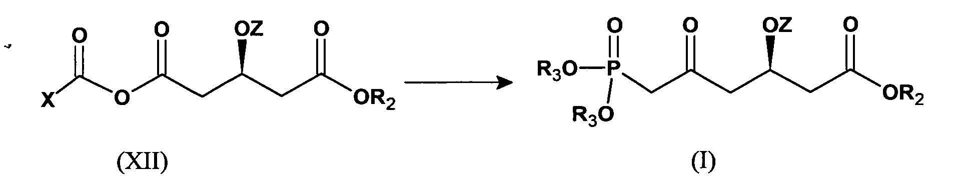 comprising reacting dialkyl phosphonate of the general formula: 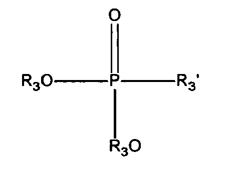 in the presence of base to obtain a phosphonium anion, wherein Z is a hydroxy protecting group; R₂ and R₃ are alkyl of 1-4 carbon atoms; X is an alkoxy group of C₁ to C₅ carbon atoms.

10. The process of claim 9, wherein all R₃ and R₃' are methyl groups.

11. The process of any of the preceding claims, wherein the base is a C₁- C₈ aryl or alkyl metal base, preferably wherein the base is an organolithium reagent, and more preferably wherein the base is phenyllithium or n-butyllithium.

12. The process of any of the preceding claims, wherein the solvent is selected from C₁-C₄ aliphatic alcoholic solvent, a C₆- C₁₀ aromatic and C₅-C₈ aliphatic hydrocarbon, a C₂-C₈ aliphatic ester, a C₄-C₈ ether (including cyclic compounds), and a C₁-C₆ aliphatic solvent with 1-4 chlorine atoms, preferably wherein the solvent is toluene, benzene, xylene, cyclohexane; ethers, methyl t-butyl ether, tetrahydrofuran or tetrahydrofuran, and more preferably wherein the solvent is tetrahydrofuran.

13. A process for preparing a statin comprising the steps of :
a) reacting a chiral base, R(+)-phenylethylamine with a compound of formula IX to obtain a salt of Formula X; 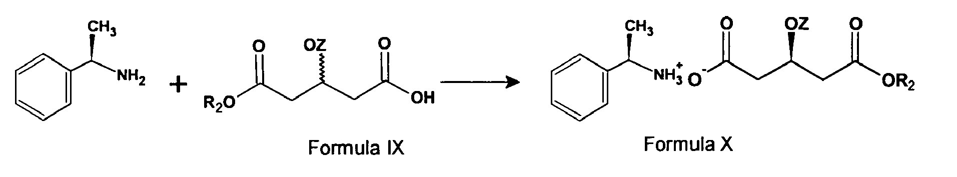
b) reacting compound X with an acid to obtain compound XI:; 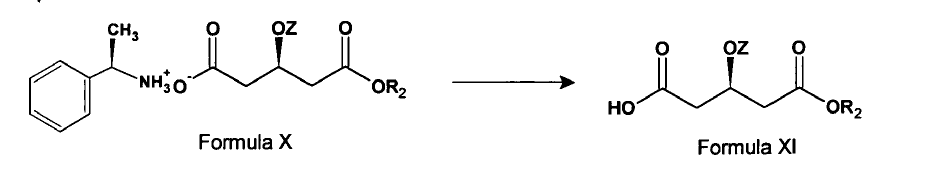
c) reacting compound formula XI with pivaloyl chloride to obtain compound of formula XII: 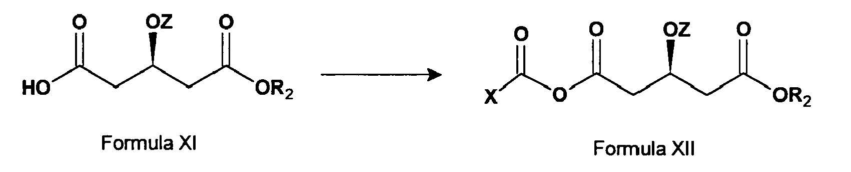
d) reacting compound formula XII with
i) a phosphonate to obtain a compound of formula I; 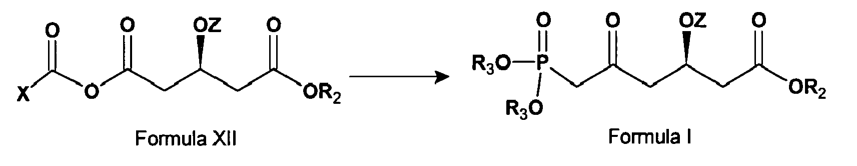
ii) or alternatively to obtain compound of Formula II 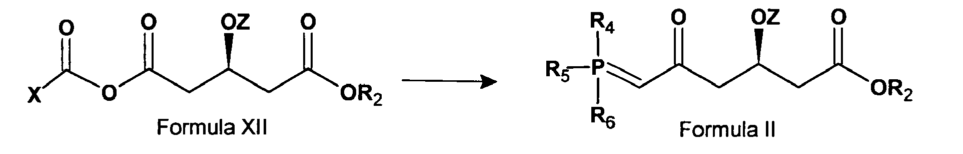
e) converting compound of Formula I or II to a statin.

14. The process of any of the preceding claims, wherein the statin is lovastatin, simvastatin, pravastatin, fluvastatin, cerivastatin, atorvastatin, rosuvastatin, and pitavastatin, and more preferably wherein the statin is rosuvastatin.

15. Use of compounds of any of the preceding claims in a process for the manufacture of a statin compound, preferably wherein the statin is lovastatin, simvastatin, pravastatin, fluvastatin, cerivastatin, atorvastatin, rosuvastatin, and pitavastatin, and more preferably rosuvastatin.
